# EUROPEAN PATENT APPLICATION

(11) **EP 4 520 768 A1**
(43) Date of publication of application: **12.03.2025**
(21) Application number: 23195818.2
(22) Date of filing: 06.09.2023
(51) Int. Cl.: C07K 16/00

(54) **VHH-CH3 FUSION PROTEINS**

(71) Applicant: BioNTech SE, 55131 Mainz (DE)
(72) Inventor: Bogen, Jan Patrick, Mainz (DE); Wüstehube-Lausch, Joycelyn, Mainz (DE); Schmoldt, Hans-Ulrich, Mainz (DE)
(74) Representative: D Young & Co LLP

(57) **Abstract**

The present invention provides improved constructs comprising immunoglobulin-domains. In particular, the present invention provides a novel VHH-CH3 fusion antigen-binding format, as well as improved methods of producing VHH-CH3 fusions.

## Description

### TECHNICAL FIELD OF THE INVENTION

The invention provides a novel format for immunoglobulin domain-containing proteins, namely single domain variable heavy immunoglobulin (VHH)-constant heavy 3 (CH3) immunoglobulin domain fusions, which comprise one or more VHH domains fused N-terminal to a CH3 immunoglobulin domain, directly or via a linking sequence. The invention also relates to improved VHH-CH3 fusions and methods of making the same, which result in reduced aggregation and improved production of the VHH-CH3 fusions.

### BACKGROUND TO THE INVENTION

Monoclonal antibodies (mAbs) are the fastest growing class of biologics with over 100 molecules approved for therapeutic usage. Despite their general favourable biophysical properties, high specificities, long half-life, and low immunogenicity, full-length mAbs fall short in various aspects. Among these are limited tumour accumulation and penetration due to their size (Yokota et al., Cancer Res., 1992, 52: 3402-8) as well as single-epitope selectivity. In the last decade, bispecific antibodies (bsAbs) aroused great interest in the scientific community with a wide variety of different formats tested for numerous applications.

In classical antibodies, the binding moiety consists of VH and VL domains, whereas camelid antibodies exhibit a unique antibody subtype comprising only a heavy chain, known as a heavy chain-only antibody (HcAb). The binding moiety in these camelid antibodies consists of a single domain, which is referred to as Variable Heavy domain of Heavy chain (VHH, also referred to herein as "single domain variable heavy immunoglobulin") domain. This single domain antibody (sdAb) is approximately the size of a human VH (~15 kDa) and, as it does not require a VL domain for antigen recognition and stable expression, it has a smaller size compared to a Fab fragment or a scFv. This smaller size permits deeper tumour penetration (Sun et al., Int J Nanomedicine, 2021, 16: 2337-2356; Jovčevska et al., BioDrugs, 2020, 34: 11-26; Chakravarty et al., Theranostics, 2014, 4: 386-98; and Kijanka et al., Nanomedicine (Lond), 2015, 10: 161-74). Some studies even report on VHHs crossing the blood brain barrier. However, similarly to fragments of conventional antibodies, VHHs as solitary binding entities are limited by their short half-life.

Conventional, full-length mAbs comprise a fragment crystallisable (Fc) region which is a homodimer of the CH2-CH3 domains. While the CH2 domain is bound by Fc gamma receptors (FcγR) and mediates the effector functions of antibodies, the recognition of the elbow regions between CH2 and CH3 by the neonatal Fc receptor (FcRn) is responsible for the long plasma half-life of IgG1 antibodies. Enormous efforts have been undertaken in order to engineer the Fc region to prolong or shorten the half-life of antibodies or to increase or silence effector functions. However, the Fc region still accounts for a large portion of the overall molecule in terms of molecular weight. This in turn limits the tumour penetration of the antibody. Various antibody formats which lack the Fc region (e.g., scFvs, diabodies, Fab, F(ab')2 and others) have been developed in order to generate a smaller binding molecule in an attempt for better tumour penetration. However, their small size also tends to result in a fast renal clearance. Hence, even though these molecules in principle may allow for deeper tumour penetrations, the limited plasma half-life hinders a strong enrichment in tumours and renders them less effective.

In an attempt to generate a molecule with an intermediate half-life and which allows for strong tumour accumulation, minibody and ΔCH2 IgGs were introduced in the 1990s. A minibody consists of a scFv which is connected to a partial hinge region of an IgG1 antibody and connected via a Glycine-Serine-Linker to the CH3 domains of an IgG1 antibody. ΔCH2 IgGs exhibit a similar architecture utilizing Fabs instead of scFvs. Both minibodies and ΔCH2 IgGs are stabilized via a (partial) hinge connecting the two heavy chains via disulphide bridges. By deleting the CH2 domain of the Fc, effector functions are abolished in these molecules. As the CH3 domain is the driver for the dimerisation in conventional Fcs, dimerisation of the two chains is not influenced. However, since FcRn binds to both CH2 and CH3, FcRn binding is abolished in these antibody formats, resulting in a reduced half-life compared to conventional, full-length mAbs. Because minibodies and ΔCH2 IgGs are still above the renal filtration size cut-off of approximately 60 kDa, their size accounts for an intermediate half-life of 20 h in humans.

These formats (minibodies and ΔCH2 IgGs) were investigated intensively in pre-clinical and clinical studies comparing them with alternative antibody formats. Even though these antibody formats showed promising (pre-)clinical profiles, their clinical usage is limited to early phase trials. One of the reasons is that scFvs, as used in minibodies, in general exhibit a strong tendency to aggregate and are therefore complicated to develop. This is underlined by the fact that of the >100 approved antibody-based therapeutics, only three (Tebentafusp, Brolucizumab and Blinatumomab) exhibit scFv moieties (The Antibody Society. Therapeutic monoclonal antibodies approved or in review in the EU or US. (29th March 2023); www.antibodysociety.org/resources/approved-antibodies).

Furthermore, it has been reported that the cysteines in the hinge region for these antibody formats (minibodies and ΔCH2 IgGs) form either the desired inter-chain disulphide (isoform A) or undesired inter-domain disulphides, i.e. intra-chain disulphides (isoform B). Hence, isoform B is only connected by non-covalent interactions between both chains, rendering it less stable compared to isoform A. These two isoforms can be distinguished in Western Blots and occur in a 50:50 ratio following antibody production (Larson et al., J. Mol. Biol., 2005, 348: 1177-90). Even though both isoforms perform similarly in *in vitro* assays, only isoform A is clinically relevant (Larson et al., J. Mol. Biol., 2005, 348: 1177-90). Therefore, additional purification steps are needed to isolate the desired isoform A, increasing the development and production costs for these antibodies.

Extensive efforts have been undertaken in order to circumvent this mispairing of disulphides. In particular, Glaser *et al*., investigated different linkers between the Fabs and the CH3 domain and found a fusion of an IgG1 hinge and the cysteine-rich IgG3 hinge (termed the "G1/G3:PAP" hinge) resulted in approximately 98% isoform A production (Glaser et al., J. Biol. Chem, 2005, 280: 41494-503). Even though these molecules exhibiting the "G1/G3:PAP" hinge mainly yield the desired inter-domain disulphide connected isoform A antibodies, it is not clear which cysteines pair inter- and which in an intra-chain manner. The authors hypothesized the formation of at least two, to a maximum of five inter-chain disulphide bridges. This unclear and probably heterogeneous disulphide linkage renders the development of these molecules as drugs complicated.

Hence, there is a need in the art for a further and improved functional antibody format with an intermediate plasma half-life, strong tumour accumulation and which is preferably suitable for large-scale manufacture for clinical use.

### SUMMARY OF THE INVENTION

The present inventors have developed an entirely novel antibody-related immunoglobulin domain-comprising class of constructs, "VHH-CH3 fusions", which comprise a polypeptide comprising one or more VHH domains fused N-terminal to a CH3 domain. The inventors have surprisingly found that these VHH-CH3 fusions provide a functional antibody format with decreased size and complexity compared to traditional antibodies, minibodies and ΔCH2 IgGs, with desirable tumour penetration and plasma half-life properties as outlined herein.

In particular, the VHH-CH3 fusions of the present invention have a decreased size compared to traditional antibodies, minibodies and ΔCH2 IgGs, while still having a size of greater than 60 kDa to prevent renal clearance. Thus, the VHH-CH3 fusions of the present invention have an optimised plasma half-life. Moreover, the optimised half-life would be expected to provide a lower anti-drug antibody response as compared to conventional antibodies due to the intermediate exposure time, as is reported in the art for minibodies.

Furthermore, the VHH-CH3 fusions of the present invention show favourable stability and no antibody effector functions (due to the absence of the CH2 domain), which is a desirable property for certain applications.

In addition, the VHH-CH3 fusions of the present invention show desirable tumour penetration and rapid tumour accumulation.

It will be appreciated that the success of the novel VHH-CH3 fusion format in the above respects was hitherto entirely unforeseeable.

Moreover, the present inventors have unexpectedly been able to overcome large-scale manufacture difficulties, and in doing so the present inventors have already provided further improved VHH-CH3 fusions and improved methods for manufacturing VHH-CH3 fusions at a large scale.

Accordingly, in a first aspect the present invention provides a construct comprising:
a) a first polypeptide comprising one or more constant immunoglobulin domains and one or more variable immunoglobulin domains N-terminal of the constant immunoglobulin domains, wherein all of the constant immunoglobulin domains in the first polypeptide are constant heavy 3 (CH3) domains, and all of the variable immunoglobulin domains N-terminal of the CH3 domains are single domain variable heavy immunoglobulin (VHH) domains.

In an embodiment, the construct further comprises:
b) a second polypeptide comprising one or more constant immunoglobulin domains and one or more variable immunoglobulin domains N-terminal of the constant immunoglobulin domain, wherein all of the constant immunoglobulin domains in the second polypeptide are CH3 domains, and all of the variable immunoglobulin domains N-terminal of the CH3 domain are VHH domains.

In some embodiments, one or more of the CH3 domains of the first polypeptide and/or one or more of the CH3 domains of the second polypeptide comprise a modification that enhances the formation of the construct comprising the first and second polypeptide.

In some embodiments, a CH3 domain of the first polypeptide forms a disulphide bond with a CH3 domain of the second polypeptide.

In some embodiments:
a) the sequence of the first polypeptide between the CH3 domains of the first polypeptide and the VHH domains located N-terminal of the CH3 domains in the first polypeptide consists of a first linking sequence; and
b) the sequence of the second polypeptide between the CH3 domains of the second polypeptide and the VHH domains located N-terminal of the CH3 domains in the second polypeptide consists of a second linking sequence.

In some embodiments, the first linking sequence does not form a disulphide bond with the second linking sequence.

In a further aspect, the invention provides one or more nucleic acid sequences capable of expressing the construct, first polypeptide or second polypeptide according to the invention.

In a further aspect, the invention provides a nucleic acid particle comprising the construct or nucleic acid sequences according to the invention. In one embodiment, the nucleic acid particle is a lipid nanoparticle (LNP).

In a further aspect, the invention provides a cell comprising the construct, nucleic acid sequences or nucleic acid particle according to the invention.

In a further aspect, the invention provides a composition comprising the construct, nucleic acid sequences, nucleic acid particle or cell according to the invention.

In a further aspect, the invention provides an *in vitro* method comprising contacting a cell with the construct, nucleic acid sequences, nucleic acid particle, cell or composition according to the invention.

In a further aspect, the invention provides the construct, nucleic acid sequences, nucleic acid particle, cell or composition according to the invention for use in a method of therapy or a diagnostic method.

In a further aspect, the invention provides the use of the construct, nucleic acid sequences, nucleic acid particle, cell or composition according to the invention for the manufacture of a medicament or diagnostic agent.

In a further aspect, the invention provides a method of therapy or a diagnostic method comprising administering the construct, nucleic acid sequences, nucleic acid particle, cell or composition according to the invention to a subject.

In a further aspect, the invention provides a method for making a construct, wherein the method comprises:
(i) providing a first polypeptide comprising one or more constant immunoglobulin domains and one or more variable immunoglobulin domains N-terminal of the constant immunoglobulin domains, wherein all of the constant immunoglobulin domains in the polypeptide are CH3 domains, wherein all of the variable immunoglobulin domains N-terminal of the CH3 domains are VHH domains, and wherein the sequence between the CH3 domains and the VHH domains located N-terminal of the CH3 domains consists of a first linking sequence;
(ii) providing a second polypeptide comprising one or more constant immunoglobulin domains and one or more variable immunoglobulin domains N-terminal of the constant immunoglobulin domains, wherein all of the constant immunoglobulin domains in the polypeptide are CH3 domains, wherein all of the variable immunoglobulin domains N-terminal of the CH3 domains are VHH domains, and wherein the sequence between the CH3 domains and the VHH domains located N-terminal of the CH3 domains consists of a second linking sequence; and
(iii) forming a construct comprising the first and second polypeptide by forming a disulphide bond between a CH3 domain of the first polypeptide and a CH3 domain of the second polypeptide, without forming a disulphide bond between the first linking sequence and the second linking sequence.

### BRIEF DESCRIPTION OF THE FIGURES

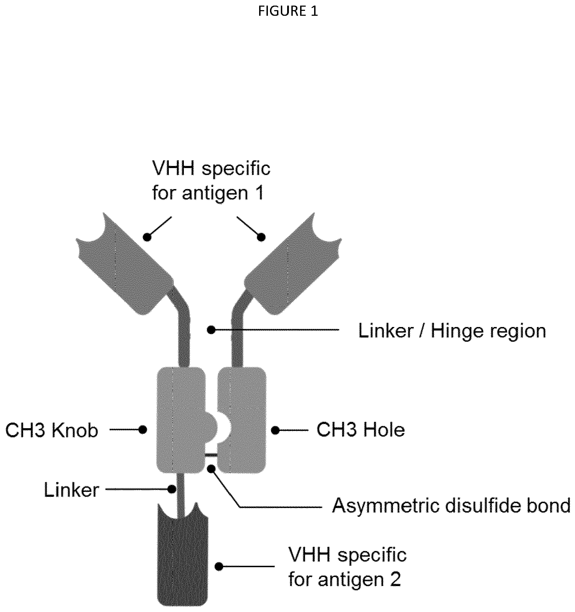

**Figure 1****: Molecular architecture of VHH-CH3 fusions.** Schematic representation of an illustrative VHH-CH3 fusion construct of the invention. The construct shown is a knob-into-hole (KiH)-based VHH-CH3 fusion, in which an additional, optional VHH domain is fused to the C-terminus of one CH3 domain in the construct.

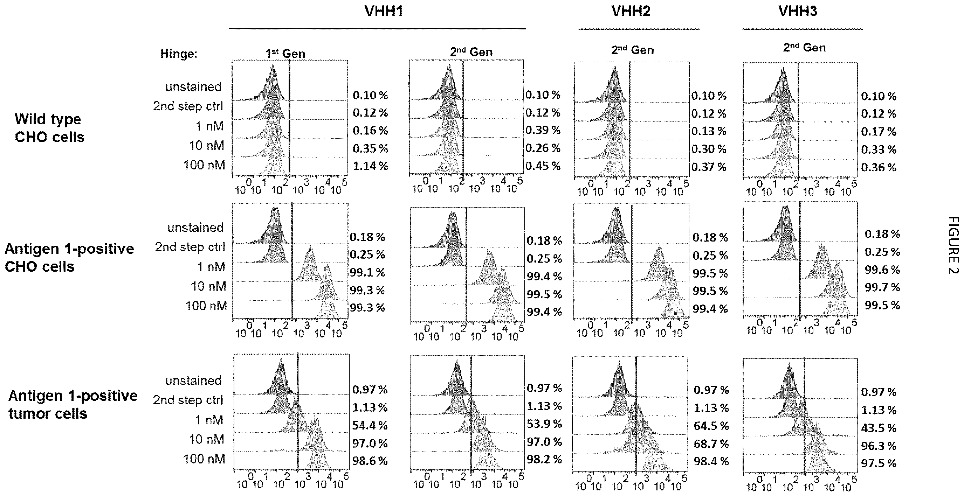

**Figure 2****: Cell binding data of VHH-CH3 fusions.** Cell binding data showing that all of the tested VHH-CH3 fusions (VHH1, VHH2 and VHH3) were fully functional, demonstrating targeting of antigen-positive tumour cells with high affinity. The top panel shows cell binding data for the wt CHO cell line (negative control), the central panel shows cell binding data for a CHO cell line expressing a tumour antigen, and the bottom panel shows cell binding data for a cancer cell line expressing the same tumour antigen. Histograms of unstained cells and cells only stained with the detection antibody are included as controls.

### DETAILED DESCRIPTION OF THE INVENTION

The present inventors have developed an entirely novel antibody-related immunoglobulin domain-comprising class of constructs, "VHH-CH3 fusions". The inventors have surprisingly found that these VHH-CH3 fusions provide a functional antibody format with both decreased size and complexity compared to minibodies and ΔCH2 IgGs. In particular, the inventors have generated an antibody fragment that combines the beneficial properties of minibodies or ΔCH2 IgGs with the small size of a VHH. To create a bispecific, trivalent construct, the inventors have also fused an additional, optional VHH domain to the C-terminus of one CH3 domain in the construct.

Moreover, the present inventors have unexpectedly been able to overcome large-scale production difficulties that may otherwise have dissuaded the skilled person from developing the VHH-CH3 fusion format. In particular, although the present inventors have now determined that it is possible to generate VHH-CH3 fusions following the teachings herein without further specific modifications to improve production, some of the VHH-CH3 fusions that were initially made tended to aggregate in a manner that was unfavourable for large-scale production. To address this, the present inventors made non-obvious and non-routine design choices, such as removing the capability of the linking sequences in the polypeptide chains of VHH-CH3 fusions to form disulphide bonds, as well as introducing a modification that enhances the dimerisation of the polypeptide chains, such as via KiH modifications and forming a disulphide bond between the CH3 domains therein.

Hence, the present inventors provide a platform technology in the form of a new antibody format for the generation at the clinical scale of small antibodies, that can be also constructed as multispecific (e.g. bispecific or trispecific) antibodies, with deep tumour penetration and intermediate plasma half-life.

### Construct

In a first aspect, the present invention provides a construct comprising:
a) a first polypeptide comprising one or more constant immunoglobulin domains and one or more variable immunoglobulin domains N-terminal of the constant immunoglobulin domains, wherein all of the constant immunoglobulin domains in the first polypeptide are constant heavy 3 (CH3) domains, and all of the variable immunoglobulin domains N-terminal of the CH3 domains are single domain variable heavy immunoglobulin (VHH) domains.

In some embodiments, the construct further comprises:
b) a second polypeptide comprising one or more constant immunoglobulin domains and one or more variable immunoglobulin domains N-terminal of the constant immunoglobulin domains, wherein all of the constant immunoglobulin domains in the second polypeptide are CH3 domains, and all of the variable immunoglobulin domains N-terminal of the CH3 domains are VHH domains.

The structure of conventional IgG molecules including VH and CH domains is well-known in the art and is reviewed, for example, by Schroeder and Cavacini (Schroeder and Cavacini, J Allergy Clin Immunol, 2010, 125: S41-S52). Unless otherwise stated, the IgG domain nomenclature is used herein.

In an embodiment, the construct is an antigen-binding fragment. In an embodiment, the construct is capable of antibody-like specific binding. In an embodiment, the construct is an antibody-like fragment. In an embodiment, the construct is capable of specific binding to an antigen. In an embodiment, the construct of the invention is an antigen-specific antibody format. In an embodiment, the construct is a VHH-CH3 fusion. VHH-CH3 fusions may also be referred to herein as "VHH-based CH3 fusions", "VHH-based minibodies" or "VHH minibodies".

As used herein, the term "antibody format" refers to a structural arrangement of a construct comprising one or more immunoglobulin domains. Said constructs may also be referred to as antigen-specific binding fragments. As used herein, terms such as "antibody-like fragment" refer to a construct comprising a portion of an antibody which retains the ability to specifically bind to a target antigen, but which has the domain presence and organisation of a VHH-CH3 fusion as defined herein. Suitably, an antibody-like fragment comprises an antigen binding site. The remainder of the construct may be any sequences which provide a suitable scaffold for the antigen binding site and display it in an appropriate manner for it to bind the antigen, within the context of the VHH-CH3 fusion format of the present invention.

As used herein, "antigen binding site" or "antigen-binding domain" means a protein or polypeptide which comprises at least one complementarity determining region (CDR). The antigen binding site may comprise 3 CDRs, i.e. be equivalent to that of a single domain antibody (sdAb) domain such as a VHH domain.

As used herein, the term "constant immunoglobulin domain" may refer to a constant domain of an immunoglobulin, e.g. a CH3 domain. Suitably, the constant immunoglobulin domain (e.g. CH3 domain) may be an IgG1, IgG2, IgG3, IgG4, IgA, IgE, IgM or IgD constant domain. Suitably, the constant immunoglobulin domain (e.g. CH3 domain) may be an IgG1, IgG2, IgG3 or IgG4 constant domain. In a preferred embodiment, the constant immunoglobulin domain (e.g. CH3 domain) is an IgG1 constant domain. Suitably, the constant immunoglobulin domain may be an IgE or IgM constant domain (e.g. CH4 domain).

As used herein, the term "variable immunoglobulin domain" may refer to a variable domain of an immunoglobulin, e.g. a VHH domain.

The immunoglobulin domains used in the invention are not limited to a specific sequence, and may comprise suitable known CH3 or VHH domains.

In a preferred embodiment, the CH3 domain is an IgG1 constant domain.

An illustrative human CH3 sequence is provided below:

GQPREPQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ ID NO:1).

In one embodiment, each of the CH3 domain of the first polypeptide and the CH3 domain of the second polypeptide comprises or consists of a sequence as set forth in SEQ ID NO: 1, or a sequence having 80%, 85%, 90%, 95%, 96%, 97%, 98%, 99% or 100% identity thereto. In an embodiment, the sequences of the CH3 domains further comprise a modification that enhances the formation of the construct comprising the first and second polypeptide as defined herein.

In an embodiment, the first polypeptide and the second polypeptide do not contain any constant immunoglobulin domain(s) other than a CH3 domain. Thus, the first polypeptide and the second polypeptide do not contain a CH1, CH2, or CH4 domain.

In a typical and highly preferred embodiment, the first polypeptide comprises a single CH3 domain and the second polypeptide, where present, comprises a single CH3 domain. Suitably, the first polypeptide may comprise a single CH3 domain. Suitably, the second polypeptide may comprise a single CH3 domain. In an embodiment, the first and/or second polypeptide each comprise one CH3 domain. In an embodiment, the first and/or second polypeptide each comprise no more than one CH3 domain. In an embodiment, the first and/or second polypeptide each comprise fewer than two CH3 domains.

In an alternative embodiment, the first and/or second polypeptide comprises more than one CH3 domain. In an embodiment, the CH3 domains of each polypeptide are adjacent, preferably immediately adjacent. In an embodiment comprising more than one CH3 domains it will be understood that, e.g., the first and/or second polypeptides do not comprise a variable domain in between the CH3 domains.

sdAbs are IgG molecules that are found naturally in e.g. camelids and sharks. sdAbs are devoid of the light chain and lack the first constant domain of the heavy chain (CH1) of conventional IgGs. Consequently, the antigen-binding fragment of sdAbs solely comprises a single variable domain, often referred to as a Variable Heavy domain of Heavy chain (VHH domain) (also referred to herein as a "single domain variable heavy immunoglobulin" domain).

In one embodiment, the first polypeptide comprises a single VHH domain N-terminal of the CH3 domains and the second polypeptide, where present, comprises a single VHH domain N-terminal of the CH3 domains. In an embodiment, the second polypeptide may comprise a single VHH domain N-terminal of the CH3 domain. In an embodiment, the first polypeptide comprises a single VHH domain N-terminal of the CH3 domain and the second polypeptide comprises a single VHH domain N-terminal of the CH3 domain.

In an embodiment, the first and/or second polypeptide comprises more than one VHH domain. In an embodiment, the first and/or second polypeptide comprises two, three, four or five VHH domains. In an embodiment, all the VHH domains in the first polypeptide have the same binding specificity. In an embodiment, all the VHH domains in the second polypeptide have the same binding specificity. In an embodiment, the VHH domains in the first polypeptide have different binding specificities. In an embodiment, the VHH domains in the second polypeptide have different binding specificities.

In one embodiment, the first and/or second polypeptide comprises two VHH domains N-terminal of the CH3 domain. In an embodiment, the first polypeptide comprises two VHH domains N-terminal of the CH3 domain. In an embodiment, the second polypeptide comprises two VHH domains N-terminal of the CH3 domain. In an embodiment, the first polypeptide comprises two VHH domains N-terminal of the CH3 domain and the second polypeptide comprises two VHH domains N-terminal of the CH3 domain.

In one embodiment, the first and the second polypeptide comprise one VHH domain N-terminal of the CH3 domain, wherein the VHH domains N-terminal of the CH3 domain of the first and the second polypeptide have the same binding specificity.

In one embodiment, the first and the second polypeptide comprise one VHH domain N-terminal of the CH3 domain, wherein the VHH domains N-terminal of the CH3 domain of the first and the second polypeptide have the different binding specificities.

In one embodiment, the first and the second polypeptide comprise one VHH domain N-terminal of the CH3 domain, wherein the VHH domains N-terminal of the CH3 domain of the first and the second polypeptide have the same binding specificity, and the first or the second polypeptide comprises one binding moiety or the cognate of a binding moiety (e.g. a VHH domain) C-terminal of the CH3 domain, wherein the binding moiety or the cognate of a binding moiety C-terminal to the CH3 domain has a different binding specificity than the VHH domains N-terminal to the CH3 domains.

In one embodiment, the first and the second polypeptide comprise one VHH domain N-terminal of the CH3 domain, wherein the VHH domains N-terminal of the CH3 domain of the first and the second polypeptide have the different binding specificities, and the first or the second polypeptide comprises one binding moiety or the cognate of a binding moiety (e.g. a VHH domain) C-terminal of the CH3 domain, wherein the binding moiety or the cognate of a binding moiety C-terminal to the CH3 domain has a different binding specificity than the VHH domains N-terminal to the CH3 domains.

In one embodiment, the first and the second polypeptide comprise one VHH domain N-terminal of the CH3 domain, wherein the VHH domains N-terminal of the CH3 domain of the first and the second polypeptide have the same binding specificity, and the first and the second polypeptide comprise one binding moiety or the cognate of a binding moiety (e.g. a VHH domain) C-terminal of the CH3 domain, wherein the binding moieties or the cognates of a binding moiety C-terminal to the CH3 domain of the first and second polypeptides have the same binding specificity, which is different from the specificity of the VHH domains N-terminal to the CH3 domains.

In one embodiment, the first and the second polypeptide comprise one VHH domain N-terminal of the CH3 domain, wherein the VHH domains N-terminal of the CH3 domain of the first and the second polypeptide have different binding specificities, and the first and the second polypeptide comprises one binding moiety or the cognate of a binding moiety (e.g. a VHH domain) C-terminal of the CH3 domain, wherein the binding moieties or the cognates of a binding moiety C-terminal to the CH3 domain of the first and second polypeptides have the same binding specificity, which is different from the specificity of the VHH domains N-terminal to the CH3 domains.

In one embodiment, the first and the second polypeptide comprise one VHH domain N-terminal of the CH3 domain, wherein the VHH domains N-terminal of the CH3 domain of the first and the second polypeptide have the same binding specificity, and the first and the second polypeptide comprise one binding moiety or the cognate of a binding moiety (e.g. a VHH domain) C-terminal of the CH3 domain, wherein the binding moieties or the cognates of a binding moiety C-terminal to the CH3 domain of the first and second polypeptides have different binding specificities, which are different from the specificity of the VHH domains N-terminal to the CH3 domains.

In one embodiment, the first and the second polypeptide comprise one VHH domain N-terminal of the CH3 domain, wherein the VHH domains N-terminal of the CH3 domain of the first and the second polypeptide have different binding specificities, and the first and the second polypeptide comprises one binding moiety or the cognate of a binding moiety (e.g. a VHH domain) C-terminal of the CH3 domain, wherein the binding moieties or the cognates of a binding moiety C-terminal to the CH3 domain of the first and second polypeptides have the different binding specificities, which are different from the specificity of the VHH domains N-terminal to the CH3 domains.

By "N-terminal of the CH3 domain" it is meant that the VHH is located N-terminal of the CH3 domain by any means known in the art. In an embodiment, the VHH is fused directly to the N-terminus of the CH3 domain, i.e. the VHH domain may be immediately adjacent to the CH3 domain. However, in an embodiment, the VHH domain is connected to the N-terminus of the CH3 domain by a linking sequence as described herein.

In an embodiment, according to the number of antigen-binding domains, e.g. VHH domains, that are present, the construct of the present invention may be monovalent, bivalent, trivalent, tetravalent or pentavalent. Similarly, in an embodiment, according to the number of different antigen-binding domains, e.g. VHH domains, that are present, the construct of the invention may be monospecific or multispecific, e.g., bispecific, trispecific, etc.

In an embodiment, in the first polypeptide, the polypeptide sequence that is C-terminal of the CH3 domains comprises a binding moiety or the cognate of a binding moiety. In an embodiment, the binding moiety or cognate is immediately C-terminal of the CH3 domains.

In an embodiment, in the first polypeptide, the polypeptide sequence that is C-terminal of the CH3 domains comprises a binding moiety. In an embodiment, the binding moiety is defined as any suitable binding entity which is capable of specifically binding to a target, such as a target polypeptide sequence. Numerous binding moieties are provided in the art, including those based on the antigen binding site of an antibody, antibody mimetics, and T-cell receptors. For example, the binding moiety may comprise: a variable immunoglobulin domain; an antigen binding site of an antibody; a single-chain variable fragment (scFv); a Fab; an F(ab)'2; an Fv; a single domain antibody (sdAb); a VHH; a single chain variable domain (which may be a VH or VL chain, having 3 CDRs); an artificial single binder such as a Darpin (designed ankyrin repeat protein); an aptamer, or an antisense oligonucleotide, e.g. a hybridisation probe. In a preferred embodiment, the binding moiety is an antigen-binding fragment of an antibody. In a preferred embodiment, the antigen-binding fragment of an antibody is a VHH.

In an embodiment, in the first polypeptide, the polypeptide sequence that is C-terminal of the CH3 domains comprises the cognate of a binding moiety. In an embodiment, the cognate of a binding moiety is defined as any motif that is specifically recognised by a binding moiety. Similarly, numerous cognates of binding moieties are provided in the art. In an embodiment, the cognate is an antigen, an epitope or a polypeptide tag that is specifically recognised by an antigen-binding fragment of an antibody. In a preferred embodiment, the cognate is a polypeptide tag. In a preferred embodiment, the cognate is a polypeptide tag that is specifically recognised by an antigen-binding fragment of an antibody. In a particularly preferred embodiment, the cognate is an ALFA tag (as described in Götzke et al., Nat Commun, 2019, 10:4403). In an embodiment, the cognate is a polypeptide comprising or consisting of the sequence SRLEEELRRRLTE (SEQ ID NO: 19).

In an embodiment, the binding moiety is an antigen-binding fragment that is capable of specifically binding to an ALFA tag. In an embodiment, the binding moiety is an antigen-binding fragment that is capable of specifically binding to a polypeptide comprising or consisting of the sequence SRLEEELRRRLTE (SEQ ID NO: 19). In an embodiment, the binding moiety is a VHH domain capable of specifically binding to the ALFA tag. In an embodiment, the binding moiety is VHH domain capable of specifically binding to a polypeptide comprising or consisting of the sequence SRLEEELRRRLTE (SEQ ID NO: 19).

In an embodiment, the binding moiety is a nanobody which specifically bind to the ALFA tag. A suitable binding moiety is described in WO 2020/053239 A1 (incorporated herein by reference).

Suitably, the binding moiety for use according to the invention may comprise the CDR1 sequence GVTISALNAMAMG (SEQ ID NO: 20) or a sequence having 1 or 2 mutations relative to said sequence, the CDR2 sequence AVSERGNAM (SEQ ID NO: 21) or a sequence having 1 or 2 mutations relative to said sequence, and the CDR3 sequence LEDRVDSFHDY (SEQ ID NO: 22) or a sequence having 1 or 2 mutations relative to said sequence. Suitably, the binding moiety for use according to the invention may comprise the CDR1 sequence GVTISALNAMAMG (SEQ ID NO: 20) or a sequence having 1 or 2 mutations relative to said sequence, the CDR2 sequence AVSSRGNAM (SEQ ID NO: 23) or a sequence having 1 or 2 mutations relative to said sequence, and the CDR3 sequence LEDRVDSFHDY (SEQ ID NO: 22) or a sequence having 1 or 2 mutations relative to said sequence. It is further understood that an antibody in which 1 or 2 mutations have been introduced to one, two, or all three of the CDR sequences is still capable of specifically binding to the ALFA tag.

Suitably, the binding moiety for use according to the invention may comprise or consist of the VHH sequence:

EVQLQESGGGLVQPGGSLRLSCTASGVTISALNAMAMGWYRQAPGERRVMVAAVSERGNAMYRESVQG
RFTVTRDFTNKMVSLQMDNLKPEDTAVYYCHVLEDRVDSFHDYWGQGTQVTVSS (SEQ ID NO:
24).

Suitably, the binding moiety for use according to the invention may comprise or consist of the VHH sequence:

EVQLQESGGGLVQPGGSLRLSCTASGVTISALNAMAMGWYRQAPGEERVMVAAVSSRGNAMYRESVQG
RFTVTRDFTNKMVSLQMDNLKPEDTAVYYCHVLEDRVDSFHDYWGQGTQVTVSS (SEQ ID NO:
25).

In an embodiment, the VHH domain(s) of the first polypeptide are specific for a first target and the VHH domain(s) of the second polypeptide are specific for a second target. In a preferred embodiment, the first and second targets are different. In an embodiment, accordingly, the construct comprising the first and second polypeptides is a heterodimer. In an alternative embodiment, the VHH domain(s) of the first polypeptide are specific for the same target as the VHH domain(s) of the second polypeptide. In an embodiment, accordingly, the construct comprising the first and second polypeptides is a homodimer.

In an embodiment, the binding moiety that is C-terminal of the CH3 domains of the first polypeptide is preferably a VHH domain and is specific for a third target. In an embodiment, the third target is different to the first and second targets. In an embodiment, the conjugate of a binding moiety that is C-terminal of the CH3 domains of the first polypeptide is not specifically recognised by any of the VHH domains of the first and second polypeptides. In an embodiment, a VHH "specific for" a target or "specifically recognising" a target refers to the VHH binds to the target with an affinity equivalent to that of a functional antibody fragment. In an embodiment, the targets are target antigens.

In an embodiment in which the first and/or second polypeptides contain a plurality of VHH domains, each VHH domain in the same polypeptide may be specific for the same target. In an embodiment in which the first and/or second polypeptides contain a plurality of VHH domains, each VHH domain in the same polypeptide may be specific for a different target.

In an embodiment, the construct comprising the first and second polypeptides may be a dimer insofar as the construct of the invention comprises two polypeptide chains (the first polypeptide and the second polypeptide) as defined herein. Accordingly, in an embodiment, the construct may further comprise additional elements such as one or more conjugates, but the construct is still considered a dimer. In an embodiment, the construct is a homodimer. In a preferred embodiment, the construct is a heterodimer.

In an embodiment, the first and second polypeptides comprise modifications that enhance the formation of a dimer, preferably a heterodimer, comprising one monomer of the first polypeptide and one monomer of the second polypeptide. In an embodiment, the modifications can be those modifications which are widely known in the art of antibody technology for the purpose of (hetero)dimerisation between antibody chains (e.g. "knobs-into-holes" (KiH) modifications). In a typical embodiment, the modifications are present in the CH3 domains of the first and second polypeptides.

In one embodiment, one or more of the CH3 domains of the first polypeptide and/or one or more of the CH3 domains of the second polypeptide comprise a modification that enhances the formation of the dimeric construct comprising the first and second polypeptide. In an embodiment wherein the first and second polypeptides each comprise more than one CH3 domain, all of the CH3 domains may comprise such modifications.

As used herein, the term "enhances the formation of the construct comprising the first and second polypeptide" includes promoting dimerisation of the first and second polypeptides. thus, in an embodiment, the modifications promote dimerisation of the first and second polypeptides. In an embodiment, the modifications promote heterodimerisation of the first and second polypeptides. By heterodimerisation it is meant that the first polypeptide is different to the second polypeptide, and the first polypeptide will form a dimer with the second polypeptide, but the first polypeptide will not form a dimer with the first polypeptide and the second polypeptide will not form a dimer with the second polypeptide. In an embodiment, the modifications promote homodimerisation of the first and second polypeptides. By homodimerisation it is meant that the first polypeptide is the same as the second polypeptide, and the first polypeptide will form a dimer with the second polypeptide.

Various means of promoting dimerisation of two domains (e.g. homo- or hetero-dimerisation) are known in the art. In an embodiment, the two domains may comprise a modification that enhances formation of dimer, e.g. a modification that increases the affinity of the two domains, promotes and/or enables the formation of one or more disulphide bonds, reduces steric hindrance to the dimerisation, promotes electrostatic and/or hydrophilic/hydrophobic interactions between the two domains, or any combination thereof.

In an embodiment, one or more of the CH3 domains of the first polypeptide and/or one or more of the CH3 domains of the second polypeptide comprise a modification that enhances the affinity between the first and second polypeptides. In an embodiment, the modification enhances the affinity between the CH3 domains of the first and second polypeptides.

In an embodiment, one or more of the CH3 domains of the first polypeptide and/or one or more of the CH3 domains of the second polypeptide comprise a modification that reduces steric hindrance to the formation of the construct comprising the first and second polypeptide. In an embodiment, the modification reduces steric hindrance between the CH3 domains of the first and second polypeptides. In an embodiment, one or more of the CH3 domains of the first polypeptide and one or more of the CH3 domains of the second polypeptide comprise a 'knobs-into-holes' modification. In an embodiment, one or more of the CH3 domains of the first polypeptide and one or more of the CH3 domains of the second polypeptide are associated via a 'knobs-into-holes' modification.

In one embodiment, one or more of the CH3 domains of the first polypeptide and/or one or more of the CH3 domains of the second polypeptide comprise a modification that promotes electrostatic interactions between the first and second polypeptide. In an embodiment, the modification promotes electrostatic interactions between the CH3 domains of the first and second polypeptides. In an embodiment, the electrostatic interactions that are promoted are favourable to the formation of a dimer (e.g. a heterodimer) comprising the first and second polypeptide.

In one embodiment, one or more of the CH3 domains of the first polypeptide and/or one or more of the CH3 domains of the second polypeptide comprise a modification that promotes hydrophilic/hydrophobic interactions between the first and second polypeptide. In an embodiment, the modification promotes hydrophilic/hydrophobic interactions between the CH3 domains of the first and second polypeptides. In an embodiment, the hydrophilic/hydrophobic interactions that are promoted are favourable to the formation of a dimer comprising the first and second polypeptide.

In an embodiment, a CH3 domain of the first polypeptide forms a disulphide bond with a CH3 domain of the second polypeptide. In a preferred embodiment, a CH3 domain of the first polypeptide forms a disulphide bond with a CH3 domain of the second polypeptide, and in addition one or more of the CH3 domains of the first polypeptide and/or one or more of the CH3 domains of the second polypeptide comprise a modification that enhances the affinity between the first and second polypeptides.

Any suitable technology for promoting the dimerisation of CH3 domains, i.e. of at least one of the CH3 domains of the first polypeptide and at least one of the CH3 domains of the second polypeptide, may be employed in the practice of the invention.

Preferably, the modification that enhances the formation of a dimer between the CH3 domains is KiH technology (Ridgeway et al., Protein Engineering, Design and Selection, 1996, 9: 617-621 and Merchant et al., Nat Biotechnol, 1998, 16: 677-681). The KiH technology is based on an engineered pair of CH3 domains that heterodimerises (CH3 heterodimer) in which asymmetric hydrophobic mutations are introduced between the homodimeric CH3 domain. KiH involves introducing mutations that create a protuberance ("knob") in the interface of the first CH3 domain and a corresponding cavity ("hole") in the interface of the second CH3 domain, such that the protuberance can be positioned in the cavity to promote heterodimer assembly and hinder homodimer formation. KiH variants therefore thermodynamically favour the formation of heterodimers rather than homodimers.

By way of further example, the present invention may employ: the strand-exchange engineered domain (SEED) CH3 dimers (Davis et al., Protein Eng Des Sel., 2010, 23:195-202); electrostatic steering employing DD-KK variants with asymmetric electrostatic interactions (Gunasekaran et al., J Biol Chem., 2010 , 285: 19637-46): Azymetric technologies by Zymeworks (https://www.zymeworks.com/technologies/azymetric/): Bispecific Engagement by Antibodies based on the T-cell receptor (BEAT) (Skegro et al., J Biol Chem., 2017, 292: 9745-9759): Fast-Ig and ART-Ig (https://www.chugai-pharm.co.jp/english/profile/rd/technologies.html): DEKK dimerisation technology (https://merus.nl/technology/multiclonics-platform/ and Nardis et al, J Biol Chem., 2017, 292: 14706-14717): HA-TF variants with asymmetric hydrophobic interactions (Moore et al., MAbs, 2011, 3: 546-57): computationally designed CH3 interfaces, such as the 7.8.60 design (Leaver-Fay et al., Structure, 2016, 24: 641-651): EW-RVT variants, which were designed to replace the conserved electrostatic interactions with asymmetric hydrophobic interactions and to add asymmetric long-range electrostatic interactions at the rim of the heterodimeric CH3 interface (Choi et al., Mol Cancer Ther., 2013, 12: 2748-59): and the K370E_{CH3A}-E357N_{CH3B} mutations employed in the "A107" variant which replaced the homodimer-favouring electrostatic interactions with heterodimer-stabilizing hydrogen bonds. Herein, EU numbering is used to denote the residues of an immunoglobulin domain (Edelman et al., Proc Natl Acad Sci U S A., 1969, 63: 78-85).

In some embodiments, one or more of the CH3 domains of the first polypeptide and one or more of the CH3 domains of the second polypeptide are associated via a KiH modification.

In some embodiments, the CH3 domain of the first polypeptide and the CH3 domain of the second polypeptide, where present, each comprise a KiH modification.

Any suitable KiH modifications known in the art may be employed in the practice of the present invention. For example, the T366W ("knob") modification and the T366S, L368A and Y407V ("hole") modifications may be used.

In an embodiment, the first polypeptide comprises the T366W modification within the CH3 domain. In an embodiment, the second polypeptide comprises the T366S, L368A and Y407V modifications within the CH3 domain. In an embodiment, the first polypeptide comprises the T366W modification within the CH3 domain and the second polypeptide comprises the T366S, L368A and Y407V modifications within the CH3 domain. In an embodiment, these modifications are according to EU residue numbering.

An illustrative CH3 domain comprising the T366W ("knob") modification (denoted in underline) is provided below:

GQPREPQVYTLPPSREEMTKNQVSLWCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 26)

An illustrative CH3 domain comprising the T366S, L368A and Y407V ("hole") modifications (each denoted in underline) is provided below:

GQPREPQVYTLPPSREEMTKNQVSLSCAVKGFYPSDIAVEWESNGQPENNYKTTPPVLDSDG
SFFLVSKLTVDKSRWQQGNVFSCSVMHEALHNHYTQKSLSLSPGK (SEQ ID NO: 27)

In an embodiment, the first polypeptide comprises a sequence as set forth in SEQ ID NO: 26, or a sequence having 80%, 85%, 90%, 95%, 96%, 97%, 98%, 99% or 100% identity thereto. In an embodiment, the second polypeptide comprises a sequence as set forth in SEQ ID NO: 27, or a sequence having 80%, 85%, 90%, 95%, 96%, 97%, 98%, 99% or 100% identity thereto. In an embodiment, the first polypeptide comprises a sequence as set forth in SEQ ID NO: 26, or a sequence having 80%, 85%, 90%, 95%, 96%, 97%, 98%, 99% or 100% identity thereto and the second polypeptide comprises a sequence as set forth in SEQ ID NO: 27, or a sequence having 80%, 85%, 90%, 95%, 96%, 97%, 98%, 99% or 100% identity thereto. In a more specific embodiment, the CH3 domains of the first and/or second polypeptides comprise or consist of the respective sequences as defined above.

In an embodiment, one or more of the CH3 domains of the first polypeptide and one or more of the CH3 domains of the second polypeptide, where present, comprise a modification to introduce a cysteine residue to promote the formation of a disulphide bond between the CH3 domains of the first and second polypeptides.

In an embodiment, the modifications to introduce a cysteine residue to promote the formation of a disulphide bond between the CH3 domains of the first and second polypeptides are configured to provide an asymmetric disulphide bond.

Suitably, the S354C mutation and the Y349C mutation described herein may be used to provide an asymmetric disulphide bond between the CH3 domains of the first and second peptide.

Accordingly, in one embodiment, the first polypeptide comprises the S354C modification within the CH3 domain. In an embodiment, the second polypeptide comprises the Y349C modification within the CH3 domain. In an embodiment, the first polypeptide comprises the S354C modification within the CH3 domain and the second polypeptide comprises the Y349C modification within the CH3 domain.

An illustrative CH3 domain comprising the S354C modification (denoted in underline) is provided below:

GQPREPQVYTLPPCREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 28)

An illustrative CH3 domain comprising the Y349C modification (denoted in underline) is provided below:

GQPREPQVCTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 29)

In an embodiment, the first polypeptide comprises a sequence as set forth in SEQ ID NO: 28, or a sequence having 80%, 85%, 90%, 95%, 96%, 97%, 98%, 99% or 100% identity thereto. In an embodiment, the second polypeptide comprises a sequence as set forth in SEQ ID NO: 29, or a sequence having 80%, 85%, 90%, 95%, 96%, 97%, 98%, 99% or 100% identity thereto. In an embodiment, the first polypeptide comprises a sequence as set forth in SEQ ID NO: 28, or a sequence having 80%, 85%, 90%, 95%, 96%, 97%, 98%, 99% or 100% identity thereto and the second polypeptide comprises a sequence as set forth in SEQ ID NO: 29, or a sequence having 80%, 85%, 90%, 95%, 96%, 97%, 98%, 99% or 100% identity thereto. In a more specific embodiment, the CH3 domains of the first and/or second polypeptides comprise or consist of the respective sequences as defined above.

In a preferred embodiment, the modification that promotes the dimerisation of the first and second polypeptides (e.g. the KiH modification) is compatible with the modification to provide a disulphide bond (e.g. an asymmetric disulphide bond) between the CH3 domains of the first and second polypeptides as described herein.

In some embodiments, one or more of the CH3 domains of the first polypeptide and one or more of the CH3 domains of the second polypeptide are associated via a KiH modification and one or more of the CH3 domains of the first polypeptide and one or more of the CH3 domains of the second polypeptide comprise a modification to introduce a cysteine residue to promote the formation of a disulphide bond between the CH3 domains of the first and second polypeptides. In an embodiment, the modifications to introduce a cysteine residue to promote the formation of a disulphide bond between the CH3 domains of the first and second polypeptides are configured to provide an asymmetric disulphide bond.

In some embodiments, the CH3 domain of the first polypeptide and the CH3 domain of the second polypeptide, where present, each comprise a KiH modification and a modification to introduce a cysteine residue to promote the formation of a disulphide bond between the CH3 domains of the first and second polypeptides. In an embodiment, the modifications to introduce a cysteine residue to promote the formation of a disulphide bond between the CH3 domains of the first and second polypeptides are configured to provide an asymmetric disulphide bond.

In an embodiment, the first polypeptide comprises the S354C and T366W modifications within the CH3 domain. In an embodiment, the second polypeptide comprises the Y349C, T366S, L368A and Y407V modifications within the CH3 domain. In an embodiment, the first polypeptide comprises the S354C and T366W modifications within the CH3 domain and the second polypeptide comprises the Y349C, T366S, L368A and Y407V modifications within the CH3 domain. In an embodiment, these modifications are according to EU residue numbering.

An illustrative CH3 domain comprising the S354C modification and T366W ("knob") modification (each denoted in underline) is provided below:

GQPREPQVYTLPPCREEMTKNQVSLWCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 2)

An illustrative CH3 domain comprising the Y349C modification and the T366S, L368A and Y407V ("hole") modifications (each denoted in underline) is provided below:

GQPREPQVCTLPPSREEMTKNQVSLSCAVKGFYPSDIAVEWESNGQPENNYKTTPPVLDSDG
SFFLVSKLTVDKSRWQQGNVFSCSVMHEALHNHYTQKSLSLSPGK (SEQ ID NO: 3)

In an embodiment, the first polypeptide comprises a sequence as set forth in SEQ ID NO: 2, or a sequence having 80%, 85%, 90%, 95%, 96%, 97%, 98%, 99% or 100% identity thereto. In an embodiment, the second polypeptide comprises a sequence as set forth in SEQ ID NO: 3, or a sequence having 80%, 85%, 90%, 95%, 96%, 97%, 98%, 99% or 100% identity thereto. In an embodiment, the first polypeptide comprises a sequence as set forth in SEQ ID NO: 2, or a sequence having 80%, 85%, 90%, 95%, 96%, 97%, 98%, 99% or 100% identity thereto and the second polypeptide comprises a sequence as set forth in SEQ ID NO: 3, or a sequence having 80%, 85%, 90%, 95%, 96%, 97%, 98%, 99% or 100% identity thereto. In a more specific embodiment, the CH3 domains of the first and/or second polypeptides comprise or consist of the respective sequences as defined above.

In some embodiments:
a) the sequence of the first polypeptide between the CH3 domains of the first polypeptide and the VHH domains located N-terminal of the CH3 domains in the first polypeptide consists of a first linking sequence; and
b) the sequence of the second polypeptide between the CH3 domains of the second polypeptide and the VHH domains located N-terminal of the CH3 domains in the second polypeptide consists of a second linking sequence.

Herein, it will be understood that in an embodiment the VHH domains N-terminal of the CH3 domains in each of the first and second polypeptide are fused directly to the respective CH3 domains. Thus, in an embodiment the VHH domains N-terminal of the CH3 domains are immediately adjacent to the CH3 domains. Thus, in an embodiment there is no other polypeptide sequence present between the VHH domains N-terminal of the CH3 domains and the CH3 domains. However, in an alternative embodiment, there is a polypeptide sequence present between the VHH domains N-terminal of the CH3 domains and the CH3 domains. In embodiments, this sequence is referred to as the linking sequence. In an embodiment where a linking sequence is not present, this may also be defined as the linking sequence comprising 0 amino acids.

Herein, the linking sequence is defined as the sequence located between the VHH domains N-terminal of the CH3 domains and the CH3 domains. The linking sequence may comprise one or more linkers (e.g. GS linkers) and/or an antibody hinge region sequence, such as those linkers and antibody hinge regions that are widely known in the art. In an embodiment, each of the linking sequences comprises a GS linker and additional sequences. In an embodiment, each of the linking sequences comprises in the following order a first GS linker, an antibody hinge region, and a second GS linker. In an embodiment, each of the linking sequences consists of a GS linker. In an embodiment, the first linking sequence is not capable of forming a disulphide bond with the second linking sequence when present in the context of the constructs of the present invention.

An illustrative linking sequence, the classical minibody-hinge region (denoted in *italics),* as described in the literature, is provided below:
*DKTHTCPPCGGGSSGGGSG* (SEQ ID NO: 4)

A further illustrative linking sequence, the hinge region of the classical ΔCH2-IgG (denoted in *italics),* as described in the literature, is provided below:
*EPKSCDKTHTCPPCGGGSSGGGSG* (SEQ ID NO: 5)

A further illustrative linking sequence, the full-length G1:G3/PAP hinge sequence (i.e. comprising the G1:G3/PAP hinge sequence (denoted in *italics)* and glycine-serine linker) as described in the literature, is provided below:
*EPKSCDKTHTCPPCPEPKSCDTPPPCPRCPAPGGGSSGGGSG* (SEQ ID NO: 6)

A further illustrative linking sequence, the "truncated G1:G3/PAP hinge sequence" (i.e. comprising a truncated G1 :G3/PAP hinge sequence (denoted in *italics)* preceded by a glycine-serine linker) is provided below:
*GGGGSGGGGSDKTHTCPPCPEPKSCDTPPPCPRCPAPGGGSSGGGSG* (SEQ ID NO: 7)

A further illustrative linking sequence, the "truncated ΔCH2-hinge" (comprising a truncated ΔCH2-hinge sequence (denoted in *italics)* preceded by a glycine-serine linker), is provided below:
*GGGGSGGGGSDKTHTCPPCGGGSSGGGSG* (SEQ ID NO: 8)

As can be seen, the truncated ΔCH2-hinge sequence shown in *italics* in SEQ ID NO: 8 above corresponds to the classical minibody-hinge sequence shown in *italics* in SEQ ID NO: 4 above. Hence, the terms "truncated ΔCH2-hinge", "minibody-hinge" and "minibody-linker" may be used herein to refer to this sequence.

A further illustrative linking sequence, the truncated ΔCH2-hinge comprising cysteine to serine modifications (i.e. the truncated ΔCH2-hinge sequence (denoted in *italics)* containing cysteine to serine modifications (denoted in underline) preceded by a glycine-serine linker), is provided below:
*GGGGSGGGGSDKTHTSPPSGGGSSGGGSG* (SEQ ID NO: 9)

A further illustrative linking sequence, the short glycine-serine linker, is provided below:
GGGGS (SEQ ID NO: 10)

In an embodiment, the linking sequence of the first and/or second polypeptide comprises or consists of a sequence as set forth in SEQ ID NO: 4, or a sequence having 80%, 85%, 90%, 95%, 96%, 97%, 98%, 99% or 100% identity thereto.

In an embodiment, the linking sequence of the first and/or second polypeptide comprises or consists of a sequence as set forth in SEQ ID NO: 5, or a sequence having 80%, 85%, 90%, 95%, 96%, 97%, 98%, 99% or 100% identity thereto.

In an embodiment, the linking sequence of the first and/or second polypeptide comprises or consists of a sequence as set forth in SEQ ID NO: 6, or a sequence having 80%, 85%, 90%, 95%, 96%, 97%, 98%, 99% or 100% identity thereto.

In a preferred embodiment, the linking sequence of the first and/or second polypeptide comprises or consists of a sequence as set forth in SEQ ID NO: 7, or a sequence having 80%, 85%, 90%, 95%, 96%, 97%, 98%, 99% or 100% identity thereto.

In a preferred embodiment, the linking sequence of the first and/or second polypeptide comprises or consists of a sequence as set forth in SEQ ID NO: 8, or a sequence having 80%, 85%, 90%, 95%, 96%, 97%, 98%, 99% or 100% identity thereto.

In a preferred embodiment, the linking sequence of the first and/or second polypeptide comprises or consists of a sequence as set forth in SEQ ID NO: 9, or a sequence having 80%, 85%, 90%, 95%, 96%, 97%, 98%, 99% or 100% identity thereto.

In a preferred embodiment, the linking sequence of the first and/or second polypeptide comprises or consists of a sequence as set forth in SEQ ID NO: 10, or a sequence having 80%, 85%, 90%, 95%, 96%, 97%, 98%, 99% or 100% identity thereto.

The first linking sequence and second linking sequence may be the same sequence or different sequences. Preferably, the first and second linking sequences are the same.

The linking sequence may include cysteine residues or be devoid of cysteine residues. Preferably, the linking sequence is devoid of cysteine residues. Thus, the first and second linking sequences within the construct may form inter-domain disulphides or may not form any inter-domain disulphide bonds. Preferably, the first and second linking sequences within the construct do not form inter-domain disulphide bonds. In a preferred embodiment, the first linking sequence does not form a disulphide bond with the second linking sequence. In particular, the linking sequences may be devoid of any cysteine residues in an embodiment wherein the CH3 domains form a disulphide bond as described herein.

The linking sequence may comprise a hinge region of an immunoglobulin or an antibody fragment. Suitably, the linking sequence may comprise the minibody/ΔCH2-hinge or the G1/G3:PAP-hinge. Suitably, the linking sequence may comprise the truncated ΔCH2-hinge or the truncated G1/G3:PAP-hinge as described herein. Suitably, the linking sequence may comprise or consist of a Glycine-Serine (GS) linker. In an embodiment, the linking sequence does not comprise a hinge region. In some embodiments, each of the first and second linking sequences consists of a Glycine-Serine (GS) linker. Suitable GS linkers for use in accordance with the invention are described elsewhere herein.

In some embodiments, each of the first and second linking sequences comprises as few as or consists of 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1, 0 amino acids.

In some embodiments, each of the first and second linking sequences comprises as few as or consists of 30, 29, 28, 27, 26, 25, 24, 23, 22, 21, 20, 19, 18, 17, 16, 15, 14, 13, 12, 11, 10, 9, 8, 7, 6, 5, 4, 3, 2, 1, 0 amino acids.

In some embodiments, each of the first and second linking sequences consists of 2 to 5 amino acids. Suitably, each of the first and second linking sequences consists of 2 amino acids. Suitably, each of the first and second linking sequences consists of 3 amino acids. Suitably, each of the first and second linking sequences consists of 4 amino acids. Suitably, each of the first and second linking sequences consists of 5 amino acids.

The VHH immunoglobulin domains used in the invention are not particularly limiting, and may comprise any antigen binding site that is specific for a selected target protein.

Methods for providing sdAbs (which comprise VHH domains) against a specific target are known in the art (see Caussinus et al.; Nat Struct Mol Biol; 2011; 19(1); 117-121 & Fulcher et al.; Open Biol; 2016; 6(10); pii 160255). Further, methods to isolate antigen-specific VHHs from immune or semisynthetic libraries using phage, yeast, or ribosome display are established in the art (see Muyldermans J Biotechnol. 2001 Jun; 74(4):277-302. & Dufner et al. Trends Biotechnol. 2006 Nov; 24(11):523-9).

By way of example, a VHH can be obtained by immunisation of e.g. dromedaries, camels, llamas or alpacas with the desired antigen and subsequent isolation of the mRNA coding for VHHs. Single domain shark variable domain of new antigen receptor (VNAR) antibodies are also known and suitable for use according to the present invention as an alternative sdAb to a VHH domain. Hence, by way of further example, a VNAR can be obtained by immunisation of sharks with the desired antigen and subsequent isolation of the mRNA coding for VNARs. Reverse transcription and PCR can then be used to generate a library of VHHs or VNARs. Standard screening techniques such as phage display and ribosome display may be used to identify the suitable clones binding the antigen of interest.

Once the most potent clones have been identified, their DNA sequence may be optimized, for example to improve their stability towards enzymes. Humanisation may also be performed.

VHHs and VNARs may be expressed in a cell using conventional vectors, such as those described herein.

The ability of the construct to specifically bind its target may be determined using methods which are known in the art. For example, determination of binding may be performed e.g. by biolayer interferometry (BLI), surface plasmon resonance (SPR) analysis (using a BIAcore instrument), western blot, flow cytometry, *in situ* hybridisation and/or microscopy. Suitably, determination of binding affinity may be performed by e.g. by biolayer interferometry (BLI), surface plasmon resonance (SPR) analysis (using a BIAcore instrument) and/or flow cytometry. Suitably, determination of binding affinity may be performed as described herein (see Example 4).

Various tumour associated antigens (TAA) are known in the art. In an embodiment, the VHH domains of the present invention are capable of specifically binding to a TAA.

### Illustrative construct sequences

Illustrative sequences of bispecific, trivalent constructs of the invention are provided below. The positions of the VHH domains within the constructs are indicated for illustrative purposes only.

### Key:

Regular font = CH3 domain
*Italics* = truncated G1:G3/PAP hinge sequence or truncated ΔCH2-hinge sequence
Underline = cysteine to serine modifications
Highlight = sequence of glycine-serine linkers

### 1) Illustrative VHH-CH3 fusion comprising a truncated G1/G3:PAP hinge and KiH modifications in the CH3 domains

### First polypeptide sequence:

[VHH1 sequence]-
GGGGSGGGGSDKTHTCPPCPEPKSCDTPPPCPRCPAPGGGSSGGGSGGQPREPQVYTLPPCR
EEMTKNQVSLWCLVKGFYPSDIAVEWESNGQPENNYKTTPPVLDSDGSFFLYSKLTVDKSRW

QQGNVFSCSVMHEALHNHYTQKSLSLSPGKGGGGSGGGS-[VHH2 sequence] (SEQ
ID NO: 11)

### Second polypeptide sequence:

[VHH3-Sequence]-
GGGGSGGGGSDKTHTCPPCPEPKSCDTPPPCPRCPAPGGGSSGGGSGGQPREPQVCTLPPSR
EEMTKNQVSLSCAVKGFYPSDIAVEWESNGQPENNYKTTPPVLDSDGSFFLVSKLTVDKSRW
QQGNVFSCSVMHEALHNHYTQKSLSLSPGK (SEQ ID NO: 12)

### 2) VHH-CH3 fusion comprising a truncated ΔCH2-hinge and KiH modifications in the CH3 domains

### First polypeptide sequence:

[VHH1 sequence]-
GGGGSGGGGSDKTHTCPPCGGGSSGGGSGGQPREPQVYTLPPCREEMTKNQVSLWCLVKGFY
PSDIAVEWESNGQPENNYKTTPPVLDSDGSFFLYSKLTVDKSRWQQGNVFSCSVMHEALHNH
YTQKSLSLSPGKGGGGSGGGS-[VHH2 sequence] (SEQ ID NO: 13)

### Second polypeptide sequence:

[VHH3 sequence]-
GGGGSGGGGSDKTHTCPPCGGGSSGGGSGGQPREPQVCTLPPSREEMTKNQVSLSCAVKGFY
PSDIAVEWESNGQPENNYKTTPPVLDSDGSFFLVSKLTVDKSRWQQGNVFSCSVMHEALHNH
YTQKSLSLSPGK (SEQ ID NO: 14)

### 3) VHH-CH3 fusion comprising a truncated ΔCH2-hinge containing cysteine to serine modifications and KiH modifications in the CH3 domains

### First polypeptide sequence:

[VHH1 sequence]-
GGGGSGGGGSDKTHTSPPSGGGSSGGGSGGQPREPQVY TLPPCREEMTKNQVSLWCLVKGFY
PSDIAVEWESNGQPENNYKTTPPVLDSDGSFFLYSKLTVDKSRWQQGNVFSCSVMHEALHNH
YTQKSLSLSPGKGGGGSGGGS-[VHH2 sequence] (SEQ ID NO: 15)

### Second polypeptide sequence:

[VHH3 sequence]-
GGGGSGGGGSDKTHTSPPSGGGSSGGGSGGQPREPQVCTLPPSREEMTKNQVSLSCAVKGFY
PSDIAVEWESNGQPENNYKTTPPVLDSDGSFFLVSKLTVDKSRWQQGNVFSCSVMHEALHNH
YTQKSLSLSPGK (SEQ ID NO: 16)

### 4) VHH-CH3 fusion comprising a short glycine-serine linker and KiH modifications in the CH3 domains

### First polypeptide sequence:

[VHH1 sequence]-
GGGGSGQPREPQVYTLPPCREEMTKNQVSLWCLVKGFYPSDIAVEWESNGQPENNYKTTPPV
LDSDGSFFLYSKLTVDKSRWQQGNVFSCSVMHEALHNHYTQKSLSLSPGKGGGGSGGGS-
[VHH2 sequence] (SEQ ID NO: 17)

### Second polypeptide sequence:

[VHH3 sequence]-
GGGGSGQPREPQVCTLPPSREEMTKNQVSLSCAVKGFYPSDIAVEWESNGQPENNYKTTPPV
LDSDGSFFLVSKLTVDKSRWQQGNVFSCSVMHEALHNHYTQKSLSLSPGK (SEQ ID NO:
18)

In one embodiment, the first polypeptide comprises a sequence as set forth in SEQ ID NO: 11, or a sequence having 80%, 85%, 90%, 95%, 96%, 97%, 98%, 99% or 100% identity thereto.

In one embodiment, the first polypeptide comprises a sequence as set forth in SEQ ID NO: 13, or a sequence having 80%, 85%, 90%, 95%, 96%, 97%, 98%, 99% or 100% identity thereto.

In one embodiment, the first polypeptide comprises a sequence as set forth in SEQ ID NO: 15, or a sequence having 80%, 85%, 90%, 95%, 96%, 97%, 98%, 99% or 100% identity thereto.

In one embodiment, the first polypeptide comprises a sequence as set forth in SEQ ID NO: 17, or a sequence having 80%, 85%, 90%, 95%, 96%, 97%, 98%, 99% or 100% identity thereto.

In one embodiment, the second polypeptide sequence comprises a sequence as set forth in SEQ ID NO: 12, or a sequence having 80%, 85%, 90%, 95%, 96%, 97%, 98%, 99% or 100% identity thereto.

In one embodiment, the second polypeptide sequence comprises a sequence as set forth in SEQ ID NO: 14, or a sequence having 80%, 85%, 90%, 95%, 96%, 97%, 98%, 99% or 100% identity thereto.

In one embodiment, the second polypeptide sequence comprises a sequence as set forth in SEQ ID NO: 16, or a sequence having 80%, 85%, 90%, 95%, 96%, 97%, 98%, 99% or 100% identity thereto.

In one embodiment, the second polypeptide sequence comprises a sequence as set forth in SEQ ID NO: 18, or a sequence having 80%, 85%, 90%, 95%, 96%, 97%, 98%, 99% or 100% identity thereto.

In one embodiment, the first polypeptide comprises a sequence as set forth in SEQ ID NO: 11, or a sequence having 80%, 85%, 90%, 95%, 96%, 97%, 98%, 99% or 100% identity thereto and the second polypeptide sequence comprises a sequence as set forth in SEQ ID NO: 12, or a sequence having 80%, 85%, 90%, 95%, 96%, 97%, 98%, 99% or 100% identity thereto.

In one embodiment, the first polypeptide comprises a sequence as set forth in SEQ ID NO: 13, or a sequence having 80%, 85%, 90%, 95%, 96%, 97%, 98%, 99% or 100% identity thereto and the second polypeptide sequence comprises a sequence as set forth in SEQ ID NO: 14, or a sequence having 80%, 85%, 90%, 95%, 96%, 97%, 98%, 99% or 100% identity thereto.

In one embodiment, the first polypeptide comprises a sequence as set forth in SEQ ID NO: 15, or a sequence having 80%, 85%, 90%, 95%, 96%, 97%, 98%, 99% or 100% identity thereto and the second polypeptide sequence comprises a sequence as set forth in SEQ ID NO: 16, or a sequence having 80%, 85%, 90%, 95%, 96%, 97%, 98%, 99% or 100% identity thereto.

In one embodiment, the first polypeptide comprises a sequence as set forth in SEQ ID NO: 17, or a sequence having 80%, 85%, 90%, 95%, 96%, 97%, 98%, 99% or 100% identity thereto and the second polypeptide sequence comprises a sequence as set forth in SEQ ID NO: 18, or a sequence having 80%, 85%, 90%, 95%, 96%, 97%, 98%, 99% or 100% identity thereto.

### Nucleic acid sequences

In a further aspect, the invention provides one or more nucleic acid sequences capable of expressing the construct according to the invention.

In a further aspect, the invention provides one or more nucleic acid sequences capable of expressing the first polypeptide sequence as defined herein.

In a further aspect, the invention provides one or more nucleic acid sequences capable of expressing the second polypeptide sequence as defined herein.

In a further aspect, the invention provides one or more nucleic acid sequences capable of expressing the first polypeptide sequence and the second polypeptide sequence as defined herein.

According to the present disclosure, terms such as "capable of expressing", "nucleic acid expressing" and "nucleic acid encoding" or similar terms are used interchangeably herein and with respect to a particular peptide or polypeptide mean that the nucleic acid, if present in the appropriate environment, e.g. within a cell, can be expressed to produce said peptide or polypeptide.

Suitably, the nucleic acid sequences capable of expressing the construct according to the invention may comprise a plurality of nucleic acid sequences which encode components of the construct such as the first polypeptide, second polypeptide, CH3 domain, VHH domain and/or linking sequence as described herein. For example, the nucleic acid sequences may comprise two, three, four or more nucleic acids which encode different components of the invention. In an embodiment, the nucleic acids comprise a first nucleic acid encoding the first polypeptide and a second nucleic acid encoding the second polypeptide.

It will be understood by the skilled person that numerous different polynucleotides and nucleic acids can encode the same polypeptide as a result of the degeneracy of the genetic code. In addition, it is to be understood that skilled persons may, using routine techniques, make nucleotide substitutions that do not affect the polypeptide sequence encoded by the polynucleotides described herein to reflect the codon usage of any particular host organism in which the polypeptides are to be expressed. Suitably, the polynucleotides of the present invention are codon optimised to enable expression in a mammalian cell, in particular a cell as described herein.

Nucleic acids according to the invention may comprise DNA and/or RNA. In an embodiment, the nucleic acids comprise DNA. In an embodiment, the nucleic acids comprise RNA. In an embodiment, the nucleic acids comprise DNA and RNA. The nucleic acids may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. Herein, it is to be understood that the polynucleotides may be modified by any method available in the art. Such modifications may be carried out in order to enhance the in vivo activity or life span of polynucleotides of interest.

In an embodiment, the one or more nucleic acid sequences of the present invention are in the form of a vector. In an embodiment, the present invention provides a vector comprising the one or more nucleic acid sequences of the present invention. Such a vector may be used to introduce the nucleic acid sequence(s) into a host cell so that it expresses a construct of the invention (suitably, a first and second polypeptide as defined herein).

In an embodiment, the vector comprises a plurality of nucleic acid sequences which encode different components as provided by the present invention. For example, in an embodiment the vector comprises a first nucleic acid sequence which encodes the first polypeptide and a second nucleic acid sequence which encodes the second polypeptide of the invention.

In embodiments, the vector may, for example, be a plasmid or a viral vector, such as a retroviral vector or a lentiviral vector, or a transposon based vector or synthetic mRNA.

In an embodiment, the vector is capable of transfecting or transducing a cell.

### Particles

The one or more nucleic acid sequences of the present invention may be present in particles comprising (i) the one or more nucleic acid sequences and (ii) at least one cationic or cationically ionizable compound such as a polymer or lipid complexing the one or more nucleic acid sequences. Electrostatic interactions between positively charged molecules such as polymers and lipids and negatively charged nucleic acid are involved in particle formation. This results in complexation and spontaneous formation of nucleic acid particles comprising the one or more nucleic acid sequences.

Accordingly, in a further aspect, the invention provides a nucleic acid particle comprising the construct or nucleic acid sequence(s) according to the invention.

Different types of nucleic acid containing particles have been described previously to be suitable for delivery of nucleic acid (e.g. RNA) in particulate form (cf., *e.g*., Kaczmarek, J. C. et al., 2017, Genome Medicine 9, 60). For non-viral delivery vehicles, nanoparticle encapsulation of nucleic acids physically protects nucleic acids from degradation and, depending on the specific chemistry, can aid in cellular uptake and endosomal escape.

In the context of the present disclosure, the term "particle" relates to a structured entity formed by molecules or molecule complexes, in particular particle forming compounds. In some embodiments, the particle contains an envelope (*e.g*., one or more layers or lamellas) made of one or more types of amphiphilic substances (*e.g*., amphiphilic lipids). In this context, the expression "amphiphilic substance" means that the substance possesses both hydrophilic and lipophilic properties. The envelope may also comprise additional substances (*e.g*., additional lipids) which do not have to be amphiphilic. Thus, the particle may be a monolamellar or multilamellar structure, wherein the substances constituting the one or more layers or lamellas comprise one or more types of amphiphilic substances (in particular selected from the group consisting of amphiphilic lipids) optionally in combination with additional substances (*e.g*., additional lipids) which do not have to be amphiphilic. In some embodiments, the term "particle" relates to a micro- or nano-sized structure, such as a micro- or nano-sized compact structure. According to the present disclosure, the term "particle" includes nanoparticles.

A "nucleic acid particle" can be used to deliver nucleic acid sequences to a target site of interest (*e.g*., cell, tissue, organ, and the like). A nucleic acid particle may be formed from lipids comprising at least one cationic or cationically ionizable lipid or lipid-like material. Without intending to be bound by any theory, it is believed that the cationic or cationically ionizable lipid or lipid-like material combines together with the nucleic acids to form aggregates, and this aggregation results in colloidally stable particles.

Nucleic acid particles (such as RNA particles and/or DNA particles) include lipid nanoparticle (LNP)-based and lipoplex (LPX)-based formulations.

In general, a lipoplex (LPX) is obtainable from mixing two aqueous phases, namely a phase comprising nucleic acid (such as RNA and/or DNA) and a phase comprising a dispersion of lipids. In some embodiments, the lipid phase comprises liposomes.

In some embodiments, liposomes are self-closed unilamellar or multilamellar vesicular particles wherein the lamellae comprise lipid bilayers and the encapsulated lumen comprises an aqueous phase. A prerequisite for using liposomes for nanoparticle formation is that the lipids in the mixture as required are able to form lamellar (bilayer) phases in the applied aqueous environment.

In some embodiments, liposomes comprise unilamellar or multilamellar phospholipid bilayers enclosing an aqueous core (also referred to herein as an aqueous lumen). They may be prepared from materials possessing polar head (hydrophilic) groups and nonpolar tail (hydrophobic) groups. In some embodiments, cationic lipids employed in formulating liposomes designed for the delivery of nucleic acids are amphiphilic in nature and consist of a positively charged (cationic) amine head group linked to a hydrocarbon chain or cholesterol derivative via glycerol.

In some embodiments, lipoplexes are multilamellar liposome-based formulations that form upon electrostatic interaction of cationic liposomes with nucleic acids (such as RNAs and/or DNAs). In some embodiments, formed lipoplexes possess distinct internal arrangements of molecules that arise due to the transformation from liposomal structure into compact nucleic acid-lipoplexes (such as RNA- and/or DNA-lipoplexes). In some embodiments, these formulations are characterized by their poor encapsulation of the nucleic acid (such as RNA) and incomplete entrapment of the nucleic acid (such as RNA).

In some embodiments, an LPX particle comprises an amphiphilic lipid, in particular cationic or cationically ionizable amphiphilic lipid, and nucleic acid (such as RNA and/or DNA, especially mRNA) as described herein. In some embodiments, electrostatic interactions between positively charged liposomes (made from one or more amphiphilic lipids, in particular cationic or cationically ionizable amphiphilic lipids) and negatively charged nucleic acid (especially mRNA) results in complexation and spontaneous formation of nucleic acid lipoplex particles. Positively charged liposomes may be generally synthesized using a cationic or cationically ionizable amphiphilic lipid, such as DOTMA and/or DODMA, and additional lipids, such as DOPE. In some embodiments, a nucleic acid (such as RNA and/or DNA, especially mRNA) lipoplex particle is a nanoparticle.

In a further aspect, the invention provides a lipoplex (LPX) comprising the construct or nucleic acid sequence(s) according to the invention.

In a further aspect, the invention provides a lipid nanoparticle (LNP) comprising the construct or nucleic acid sequence(s) according to the invention.

In general, a lipid nanoparticle (LNP) is obtainable from direct mixing of nucleic acid (such as RNA and/or DNA) in an aqueous phase with lipids in a phase comprising an organic solvent, such as ethanol. In that case, lipids or lipid mixtures can be used for particle formation, which do not form lamellar (bilayer) phases in water.

In some embodiments, LNPs comprise or consist of a cationic/ionisable lipid and helper lipids such as phospholipids, cholesterol, and/or polyethylene glycol (PEG) lipids. In some embodiments, in the nucleic acid LNPs (such as DNA LNPs) described herein the nucleic acid (such as DNA) is bound by ionisable lipid that occupies the central core of the LNP. In some embodiments, PEG lipid forms the surface of the LNP, along with phospholipids. In some embodiments, the surface comprises a bilayer. In some embodiments, cholesterol and ionisable lipid in charged and uncharged forms can be distributed throughout the LNP.

In some embodiments, nucleic acid (such as RNA and/or DNA, *e.g*., mRNA) may be noncovalently associated with a particle as described herein. In embodiments, the nucleic acid (such as RNA and/or DNA, especially mRNA) may be adhered to the outer surface of the particle (surface nucleic acid) and/or may be contained in the particle (encapsulated nucleic acid (such as encapsulated DNA).

In some embodiments, the particles (e.g., LNPs and LPXs) described herein have a size (such as a diameter) in the range of about 10 to about 2000 nm, such as at least about 15 nm (e.g., at least about 20 nm, at least about 25 nm, at least about 30 nm, at least about 35 nm, at least about 40 nm, at least about 45 nm, at least about 50 nm, at least about 55 nm, at least about 60 nm, at least about 65 nm, at least about 70 nm, at least about 75 nm, at least about 80 nm, at least about 85 nm, at least about 90 nm, at least about 95 nm, or at least about 100 nm) and/or at most 1900 nm (e.g., at most about 1900 nm, at most about 1800 nm, at most about 1700 nm, at most about 1600 nm, at most about 1500 nm, at most about 1400 nm, at most about 1300 nm, at most about 1200 nm, at most about 1100 nm, at most about 1000 nm, at most about 950 nm, at most about 900 nm, at most about 850 nm, at most about 800 nm, at most about 750 nm, at most about 700 nm, at most about 650 nm, at most about 600 nm, at most about 550 nm, or at most about 500 nm), such as in the range of about 20 to about 1500 nm, such as about 30 to about 1200 nm, about 40 to about 1100 nm, about 50 to about 1000 nm, about 60 to about 900 nm, about 70 to 800 nm, about 80 to 700 nm, about 90 to 600 nm, or about 50 to 500 nm or about 100 to 500 nm, such as in the range of 10 to 1000 nm, 15 to 500 nm, 20 to 450 nm, 25 to 400 nm, 30 to 350 nm, 40 to 300 nm, 50 to 250 nm, 60 to 200 nm, or 70 to 150 nm.

In some embodiments, the particles described herein are nanoparticles. The term "nanoparticle" relates to a nano-sized particle comprising nucleic acid (especially DNA) as described herein and at least one cationic or cationically ionisable lipid, wherein all three external dimensions of the particle are in the nanoscale, *i.e*., at least about 1 nm and below about 1000 nm. Preferably, the size of a particle is its diameter.

### Cell

In a further aspect, the invention provides a cell comprising the construct, nucleic acid sequence(s) or LNP according to the invention.

Methods for engineering such cells are known in the art and include but are not limited to genetic modification of cells e.g. by transduction such as retroviral or lentiviral transduction, transfection (such as transient transfection - DNA or RNA based) including lipofection, polyethylene glycol, calcium phosphate and electroporation. Any suitable method may be used to introduce a nucleic acid sequence into a cell.

A cell of the present invention may be generated by introducing DNA or RNA coding for the construct as defined herein by one of many means including transduction with a viral vector, transfection with DNA or RNA.

The cell of the invention may be made by introducing to a cell (e.g. by transduction or transfection) the nucleic acid sequence according to the present invention.

The cell of the invention may secrete the construct of the invention, carry the construct of the invention on the cell surface, or both. Preferably, the cell of the invention secretes the construct of the invention and carries the construct of the invention on the cell surface.

### In vitro method

In a further aspect, the invention provides an *in vitro* method comprising contacting a cell with the construct, nucleic acid sequence(s), LNP or composition according to the invention. In an embodiment, the cell that is contacted is a patient, sample or subject cell. It is to be understood that the cell that is contacted is not necessarily a cell of the present invention.

The constructs of the invention may be employed in any method known in the art which utilises antibodies and/or antibody fragments. For example, the bispecific constructs of the invention may be employed as an immune cell engager (e.g. for T cells, NK cells, myeloid cells, and the like) to facilitate crosslinking of two cells, in receptor activation or inactivation, crosslinking of proteins on surface of the same cell or on the surface of two different cells, transport through the blood brain barrier, and for conjugation to a drug (i.e. as an antibody-drug conjugate).

### Composition

In a further aspect, the invention provides a composition comprising the construct, nucleic acid sequence(s), LNP or cell according to the invention.

The composition may be a pharmaceutical composition.

The pharmaceutical composition may additionally comprise a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

### Therapeutic use

In a further aspect, the invention provides the construct, nucleic acid sequence(s), LNP, cell or composition according to the invention for use in a method of therapy or a diagnostic method.

In a further aspect, the invention provides the use of the construct, nucleic acid sequence(s), LNP, cell or composition according to the invention for the manufacture of a medicament or diagnostic agent.

In a further aspect, the invention provides a method of therapy or a diagnostic method comprising administering the construct, nucleic acid sequence(s), LNP, cell or composition according to the invention to a subject.

A method for therapy includes a method for treating a disease as well as a method for preventing a disease.

A method for treating a disease relates to the therapeutic use of the construct, nucleic acid sequence(s), LNP, cell or composition according to the invention. In this respect, the construct, nucleic acid sequence(s), LNP, cell or composition according to the invention may be administered to a subject having an existing disease or condition in order to lessen, reduce or improve at least one symptom associated with the disease and/or to slow down, reduce or block the progression of the disease.

The method for preventing a disease relates to the prophylactic use of the construct, nucleic acid sequence(s), LNP, cell or composition according to the invention. In this respect, the construct, nucleic acid sequence(s), LNP, cell or composition according to the invention may be administered to a subject who has not yet contracted the disease and/or who is not showing any symptoms of the disease to prevent or impair the cause of the disease or to reduce or prevent development of at least one symptom associated with the disease. The subject may have a predisposition for, or be thought to be at risk of developing, the disease.

Suitably, the vector(s) or nucleic acids may be introduced by transduction. Suitably, the vector(s) or nucleic acids may be introduced by transfection.

The disease to be treated and/or prevented may be cancer.

Suitably, the cancer may be a solid tumour or a liquid tumour.

The cancer may be a cancer such as neuroblastoma, prostate cancer, bladder cancer, breast cancer, colon cancer, endometrial cancer, kidney cancer (renal cell), leukaemia, lung cancer, melanoma, non-Hodgkin lymphoma, pancreatic cancer, and thyroid cancer.

The cell of the present invention may be capable of killing target cells, such as cancer cells. The target cell may be recognisable by expression of a TAA. In one such embodiment, one or more of the VHH domains of the construct of the invention is capable of specifically binding to a TAA.

### Method for making a construct

In a further aspect, the invention provides a method for making a construct, wherein the method comprises:
(i) providing a first polypeptide comprising one or more constant immunoglobulin domains and one or more variable immunoglobulin domains N-terminal of the constant immunoglobulin domains, wherein all of the constant immunoglobulin domains in the polypeptide are CH3 domains, wherein all of the variable immunoglobulin domains N-terminal of the CH3 domains are VHH domains, and wherein the sequence between the CH3 domains and the VHH domains located N-terminal of the CH3 domains consists of a first linking sequence;
(ii) providing a second polypeptide comprising one or more constant immunoglobulin domains and one or more variable immunoglobulin domains N-terminal of the constant immunoglobulin domains, wherein all of the constant immunoglobulin domains in the polypeptide are CH3 domains, wherein all of the variable immunoglobulin domains N-terminal of the CH3 domains are VHH domains, and wherein the sequence between the CH3 domains and the VHH domains located N-terminal of the CH3 domains consists of a second linking sequence; and
(iii) forming a construct comprising the first and second polypeptide by forming a disulphide bond between a CH3 domain of the first polypeptide and a CH3 domain of the second polypeptide, without forming a disulphide bond between the first linking sequence and the second linking sequence.

The construct, first polypeptide, second polypeptide and linking sequences of this method are those of the present invention.

In an embodiment, step (iii) further involves the association of a CH3 domain of the first polypeptide with a CH3 domain of the second polypeptide via KiH modifications.

In an embodiment, the method comprises providing the first polypeptide and the second polypeptide at a ratio of 1:1, 1:2, 1:3, 1:4 or 1:5 respectively. Preferably, the ratio is at least 1:2. More preferably the ratio is at least 1:3. Suitably, the ratio is at least 1:5. In preferred embodiments of this type, the first polypeptide comprises one or more "knobs" modifications and the second polypeptide comprises one or more "holes" modifications.

### General definitions

It is to be understood herein that references to any feature in respect of the 'first polypeptide "and/or" the second polypeptide', or the 'first polypeptide "and, where present," the second polypeptide' have the same meaning, and include independent selections of a) the feature in question applying to the first polypeptide alone; b) the feature in question applying to the second polypeptide alone, and c) the feature in question applying to both the first and second polypeptides. For example, a statement of "wherein the first polypeptide comprises a single VHH domain and the second polypeptide, where present, comprises a single VHH domain", refers to embodiments wherein explicitly a) the first polypeptide comprises a single VHH domain, b) the second polypeptide comprises a single VHH domain and c) the first and second polypeptides each comprise a single VHH domain. It will be understood that each of these embodiments may apply to the constructs and polypeptides of the present invention as appropriate.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino (N) to carboxy (C) orientation, respectively.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

The term "polypeptide" is used in the conventional sense to mean a series of amino acids, typically L-amino acids, connected one to the other, typically by peptide bonds between the α-amino and carboxyl groups of adjacent amino acids. The term "polypeptide" is used interchangeably with the terms "amino acid sequence", "peptide" and/or "protein". The term "residues" is used to refer to amino acids in an amino acid sequence.

The term "variant" in relation to a polypeptide refers to a polypeptide that has an equivalent function to the amino acid sequences described herein, but which includes one or more amino acid substitutions, insertions or deletions.

As used herein, the terms "polynucleotide", "nucleotide", "nucleic acid sequence" and "nucleic acid" are intended to be synonymous with each other.

The terms "variant", "homologue" or "derivative" in relation to a nucleotide sequence include any substitution of, variation of, modification of, replacement of, deletion of or addition of one (or more) nucleic acid from or to the sequence.

"Sequence identity" between two nucleic acid sequences indicates the percentage of nucleotides that are identical between the sequences. The terms "% identical" and "% identity" or similar terms are intended to refer, in particular, to the percentage of nucleotides or amino acids which are identical in an optimal alignment between the sequences to be compared. Said percentage is purely statistical, and the differences between the two sequences may be but are not necessarily randomly distributed over the entire length of the sequences to be compared. Comparisons of two sequences are usually carried out by comparing the sequences, after optimal alignment, with respect to a segment or "window of comparison", in order to identify local regions of corresponding sequences. The optimal alignment for a comparison may be carried out manually or with the aid of the local homology algorithm by Smith and Waterman, 1981, Ads App. Math. 2, 482, with the aid of the global homology algorithm by Needleman and Wunsch, 1970, J. Mol. Biol. 48, 443, with the aid of the similarity search algorithm by Pearson and Lipman, 1988, Proc. Natl Acad. Sci. USA 88, 2444, or with the aid of computer programs using said algorithms (GAP, BESTFIT, FASTA, BLAST P, BLAST N and TFASTA in Wisconsin Genetics Software Package, Genetics Computer Group, 575 Science Drive, Madison, Wis.). In some embodiments, percent identity of two sequences is determined using the BLASTN or BLASTP algorithm, as available on the United States National Center for Biotechnology Information (NCBI) website (e.g., at blast.ncbi.nlm.nih.gov/Blast.cgi?PAGE_TYPE=BlastSearch&BLAST_SPEC=blast2seq&LINK _LOC=align2seq). In some embodiments, the algorithm parameters used for BLASTN algorithm on the NCBI website include: (i) Expect Threshold set to 10; (ii) Word Size set to 28; (iii) Max matches in a query range set to 0; (iv) Match/Mismatch Scores set to 1, -2; (v) Gap Costs set to Linear; and (vi) the filter for low complexity regions being used. In some embodiments, the algorithm parameters used for BLASTP algorithm on the NCBI website include: (i) Expect Threshold set to 10; (ii) Word Size set to 3; (iii) Max matches in a query range set to 0; (iv) Matrix set to BLOSUM62; (v) Gap Costs set to Existence: 11 Extension: 1; and (vi) conditional compositional score matrix adjustment.

Percentage identity is obtained by determining the number of identical positions at which the sequences to be compared correspond, dividing this number by the number of positions compared (e.g., the number of positions in the reference sequence) and multiplying this result by 100.

In some embodiments, the degree of similarity or identity is given for a region which is at least about 50%, at least about 60%, at least about 70%, at least about 80%, at least about 90% or about 100% of the entire length of the reference sequence. For example, if the reference nucleic acid sequence consists of 200 nucleotides, the degree of identity is given for at least about 100, at least about 120, at least about 140, at least about 160, at least about 180, or about 200 nucleotides, in some embodiments continuous nucleotides. In some embodiments, the degree of similarity or identity is given for the entire length of the reference sequence.

In some embodiments, "isolated" means removed (e.g., purified) from the natural state or from an artificial composition, such as a composition from a production process. For example, a nucleic acid, peptide or polypeptide naturally present in a living animal is not "isolated", but the same nucleic acid, peptide or polypeptide partially or completely separated from the coexisting materials of its natural state is "isolated". An isolated nucleic acid, peptide or polypeptide can exist in substantially purified form, or can exist in a non-native environment such as, for example, a host cell.

The term "expression" as used herein is defined as the transcription and/or translation of a particular nucleotide sequence.

In the context of the present disclosure, the term "transcription" relates to a process, wherein the genetic code in a DNA sequence is transcribed into RNA (especially mRNA). Subsequently, the RNA may be translated into peptide or polypeptide.

With respect to RNA, the term "expression" or "translation" relates to the process in the ribosomes of a cell by which a strand of mRNA directs the assembly of a sequence of amino acids to make a peptide or polypeptide.

The term "nucleic acid" comprises deoxyribonucleic acid (DNA), ribonucleic acid (RNA), combinations thereof, and modified forms thereof. The term comprises genomic DNA, cDNA, mRNA, recombinantly produced and chemically synthesized molecules. A nucleic acid may be present as a single-stranded or double-stranded and linear or covalently circularly closed molecule. A nucleic acid can be isolated. The term "isolated nucleic acid" means, according to the present disclosure, that the nucleic acid (i) was amplified in vitro, for example via polymerase chain reaction (PCR) for DNA or in vitro transcription (using, e.g., an RNA polymerase) for RNA, (ii) was produced recombinantly by cloning, (iii) was purified, for example, by cleavage and separation by gel electrophoresis, or (iv) was synthesized, for example, by chemical synthesis.

The term "nucleoside" (abbreviated herein as "N") relates to compounds which can be thought of as nucleotides without a phosphate group. While a nucleoside is a nucleobase linked to a sugar (e.g., ribose or deoxyribose), a nucleotide is composed of a nucleoside and one or more phosphate groups. Examples of nucleosides include cytidine, uridine, pseudouridine, adenosine, and guanosine.

The five standard nucleosides which usually make up naturally occurring nucleic acids are uridine, adenosine, thymidine, cytidine and guanosine. The five nucleosides are commonly abbreviated to their one letter codes U, A, T, C and G, respectively. However, thymidine is more commonly written as "dT" ("d" represents "deoxy") as it contains a 2'-deoxyribofuranose moiety rather than the ribofuranose ring found in uridine. This is because thymidine is found in deoxyribonucleic acid (DNA) and not ribonucleic acid (RNA). Conversely, uridine is found in RNA and not DNA. The remaining three nucleosides may be found in both RNA and DNA. In RNA, they would be represented as A, C and G, whereas in DNA they would be represented as dA, dC and dG.

A modified purine (A or G) or pyrimidine (C, T, or U) base moiety is preferably modified by one or more alkyl groups, more preferably one or more C1-4 alkyl groups, even more preferably one or more methyl groups. Particular examples of modified purine or pyrimidine base moieties include N7-alkyl-guanine, N6-alkyl-adenine, 5-alkyl-cytosine, 5-alkyl-uracil, and N(1)-alkyl-uracil, such as N7-C1-4 alkyl-guanine, N6-C1-4 alkyl-adenine, 5-C1-4 alkyl-cytosine, 5-C1-4 alkyl-uracil, and N(1)-C1-4 alkyl-uracil, preferably N7-methyl-guanine, N6-methyl-adenine, 5-methyl-cytosine, 5-methyl-uracil, N1-methyl-pseudouridine, and N(1)-methyl-uracil.

Herein, the term "DNA" relates to a nucleic acid molecule which includes deoxyribonucleotide residues. In preferred embodiments, the DNA contains all or a majority of deoxyribonucleotide residues. As used herein, "deoxyribonucleotide" refers to a nucleotide which lacks a hydroxyl group at the 2'-position of a β-D-ribofuranosyl group. DNA encompasses without limitation, double stranded DNA, single stranded DNA, isolated DNA such as partially purified DNA, essentially pure DNA, synthetic DNA, recombinantly produced DNA, as well as modified DNA that differs from naturally occurring DNA by the addition, deletion, substitution and/or alteration of one or more nucleotides. Such alterations may refer to addition of non-nucleotide material to internal DNA nucleotides or to the end(s) of DNA. It is also contemplated herein that nucleotides in DNA may be non-standard nucleotides, such as chemically synthesized nucleotides or ribonucleotides. For the present disclosure, these altered DNAs are considered analogs of naturally-occurring DNA. A molecule contains "a majority of deoxyribonucleotide residues" if the content of deoxyribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

DNA may be recombinant DNA and may be obtained by cloning of a nucleic acid, in particular cDNA. The cDNA may be obtained by reverse transcription of RNA.

The term "RNA" relates to a nucleic acid molecule which includes ribonucleotide residues. In preferred embodiments, the RNA contains all or a majority of ribonucleotide residues. As used herein, "ribonucleotide" refers to a nucleotide with a hydroxyl group at the 2'-position of a β-D-ribofuranosyl group. RNA encompasses without limitation, double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations may refer to addition of non-nucleotide material to internal RNA nucleotides or to the end(s) of RNA. It is also contemplated herein that nucleotides in RNA may be non-standard nucleotides, such as chemically synthesized nucleotides or deoxynucleotides. For the present disclosure, these altered/modified nucleotides can be referred to as analogs of naturally occurring nucleotides, and the corresponding RNAs containing such altered/modified nucleotides (i.e., altered/modified RNAs) can be referred to as analogs of naturally occurring RNAs. A molecule contains "a majority of ribonucleotide residues" if the content of ribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

"RNA" includes mRNA, tRNA, ribosomal RNA (rRNA), small nuclear RNA (snRNA), self-amplifying RNA (saRNA), single-stranded RNA (ssRNA), dsRNA, inhibitory RNA (such as antisense ssRNA, small interfering RNA (siRNA), or microRNA (miRNA)), activating RNA (such as small activating RNA) and immunostimulatory RNA (isRNA). In some embodiments, "RNA" refers to mRNA.

It must be noted that as used herein and in the appended claims, the singular forms "a", "an", and "the" include plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of" also include the term "consisting of'.

The publications discussed herein are provided solely for their disclosure prior to the filing date of the present application. Nothing herein is to be construed as an admission that such publications constitute prior art to the claims appended hereto.

The invention will now be further described by way of Examples, which are meant to serve to assist one of ordinary skill in the art in carrying out the invention and are not intended in any way to limit the scope of the invention.

### EXAMPLES

### Materials & Methods

### Expression of recombinant VHH-CH3 fusion proteins

Human codon optimized VHH-fusion sequences were generated by gene synthesis and cloned into pTWIST expression vector (Twist Bioscience). For recombinant expression of the VHH-CH3 fusion, Expi293 cells were transfected with ExpiFectamine 293 (ThermoFisher Scientific) using manufactures protocol. Culture supernatants were harvested from Expi293 producer cell lines after 4-5 days, centrifuged and recombinant proteins were used for further characterization and purification.

### Western Blot for Aggregate detection

For detection of aggregation in cell culture supernatant, a Western Blot analysis was performed. Cell culture supernatant was mixed with non-reducing Lämmli buffer and subsequently applied to an "Any kD Criterion TGX Stain-Free" gel from Bio-Rad. The SDS-PAGE was loaded with 10-20 µL of a 10-fold diluted supernatant sample and run at 250 V for 40-80 min on ice. Subsequently, the gel was washed in water and transferred onto a nitrocellulose membrane using the Trans-Blot Turbo System of Bio-Rad. The blotted membrane was washed with PBS-T and blocked with 5% milk powder for 1 h at room temperature. After further washing steps and incubation of an anti-VHH-HRP or an antihuman-HRP detection antibody overnight, further wash steps were performed. The HRP substrate was applied, and the Western Blot was analysed using a Bio-Rad ChemiDoc system. Aggregates are defined as molecular weights above the expected molecular size of the protein of interest.

### Affinity to Antigen 1

For affinity measurements an Octet HTX device from Sartorius was used.

Commercially available SAX2.0 biosensors were soaked for at least 10 min in kinetic buffer (KB) purchased from the instrument manufacturer. Following a 60 sec baseline in KB, the biotinylated antigen 2 was loaded for 240 sec. Subsequently, a quenching step in a 100 µg/mL biocytin solution was performed for 120 sec, followed by a 120 sec baseline in KB.

The VHH-CH3 fusion was applied to all biosensors for 300 sec or until a threshold of 0.7 nm in response was reached. Following a 60 sec baseline in KB, association to antigen 1 was measured over 600 sec using seven different concentrations of the antigen 1 starting at 50 nM in a 1:1 serial dilution series down to 0.78125 nM. As a reference, KB without the antigen 1 was measured. The dissociation was acquired over 600 sec in KB. The signal of the reference well was subtracted from the signal of all other biosensors and the signals of these processed data were aligned to the average of the baseline before the association step. For inter-step correction, the data were aligned to the dissociation step and Savitzky-Golay filtering was applied to all curves. Association and dissociations were globally fitted using a 1:1 Langmuir binding model.

### Affinity to Antigen 2

For affinity measurements an Octet HTX device from Sartorius was used.

Commercially available SAX2.0 biosensors were soaked for at least 10 min in kinetic buffer (KB) purchased from the instrument manufacturer. Following a 60 sec baseline in KB, the biotinylated antigen 2 was loaded for 240 sec. Subsequently, a quenching step in a 100 µg/mL biocytin solution was performed for 120 sec, followed by a 120 sec baseline in KB.

Association was measured over 900 sec using seven different concentrations of a VHH-CH3 fusion starting at 10 nM in a 1:1 serial dilution series down to 156.25 pM. As a reference, KB without the VHH-CH3 was measured. The dissociation was acquired over 1500 sec in KB. The signal of the reference well was subtracted from the signal of all other biosensors and the signals of these processed data were aligned to the average of the second baseline. For inter-step correction, the data were aligned to the dissociation step and Savitzky-Golay filtering was applied to all curves. Association and dissociations were globally fitted using a 1:1 Langmuir binding model.

### DLS and NanoDSF

Thermal stabilities (via NanoDSF) and size distribution (via DLS) were investigated using the Prometheus PANTA from NanoTemper. 10 µL of VHH-CH3 fusion protein solution was loaded into capillaries. After loading, the capillaries were mounted into the instrument and the DLS analysis was performed. To assess thermal stability, a heat ramp of 1°C/min from 25°C to 95°C was subsequently applied to all samples. During this process the intrinsic fluorescence of the proteins was measured at 350 nm and 330 nm. The ratio was plotted against the temperature and the first derivative was calculated. Minima and maxima corresponded to the TM values.

### SEC

Aggregation analysis was performed via SEC utilizing an Agilent Infinity II HPLC and a Biozen 1.8 µm dSEC-2, 200 Å LC column (300 × 4.6 mm). Flowrates were adjusted to 0.25 mL/min, resulting in approximately 255 bar pressure. As mobile phase 0.2 M potassium phosphate, 250 mM KCI, pH 6.2, 5% acetonitrile was used. Each run took 20 min excluding a 2-3 min wash step between each analysis. 10 µL of the VHH-CH3 fusion proteins were applied and detected by absorption at 280 nm.

### Flow cytometry

Antigen 1-positive or -negative cells were seeded in a 96-well microtiter plate (round button) to a final density of 2×10⁵ and incubated in the presence of the VHH-CH3 fusion for 30 min at 4°C. Afterwards, cells were washed twice with FACS-buffer (1x phosphate-buffer saline + 5 mL 0.5 M ethylenediaminetetraacetic acid + 10 mL Fetal Bovine Serum (FBS)) and once with 1x phosphate-buffer saline and subsequently incubated with a AF647-tagged antigen 2 for 30 min at 4°C. After another washing step with FACS-buffer cells were fixated with BD Fixative and analysed with the FACS celesta (BD Biosciences). Excitation laser Line 633 nm was used to detect the AF647 signals.

### Example 1 - Construction of VHH-CH3 fusions

The present inventors elucidated for the first time the utilisation of VHH-CH3 fusions as a "minibody-like" antibody format. Furthermore, the present inventors investigated for the first time a knob-into-hole (KiH)-based VHH-derived CH3 fusion as an antibody format (e.g. for trivalent, bispecific KiH-based antibodies). In particular, to create a bispecific, trivalent antibody the present inventors fused an additional VHH to the C-terminus of one CH3 domain (Figure 1). To force heterodimerisation of the respective CH3 domains, the Knob-into-hole (KiH) technology was implemented. Hence, CH3 domains comprising sequences as set forth in SEQ ID NOs: 2 and 3 were used.

The present inventors first generated ΔCH2-IgGs for comparison. ΔCH2-IgGs were generated with either the clinically validated ΔCH2-hinge (SEQ ID NO: 5) or the G1/G3:PAP hinge (SEQ ID NO: 6). The hinge regions used (i.e. as set forth in SEQ ID NO:5 and SEQ ID NO: 6) comprise the sequence EPKSC (SEQ ID NO: 30) to connect with the light chain within the Fab of the ΔCH2-IgGs. Each construct contained an additional VHH fused to the C-terminus of one CH3 domain, and KiH modifications in the CH3 domains to force heterodimerisation.

The present inventors then performed analysis of aggregation by Western Blot using non-reduced samples. Western blot analysis of ΔCH2-IgGs (for comparison) utilizing either the ΔCH2-hinge (SEQ ID NO: 5) or the G1/G3:PAP-hinge (SEQ ID NO: 6) showed no aggregation for both constructs, and the products were produced in high yield.

The present inventors next generated VHH-based CH3 fusions (according to the present invention) with either the truncated ΔCH2-hinge (SEQ ID NO: 8) or the truncated G1/G3:PAP hinge (SEQ ID NO: 7). The truncated ΔCH2-hinge (SEQ ID NO: 8) and truncated G1/G3:PAP hinge (SEQ ID NO: 7) sequences correspond to the ΔCH2-hinge (SEQ ID NO: 5) and G1/G3:PAP hinge (SEQ ID NO: 6) regions used for the ΔCH2-IgGs with the exception that the EPKSC sequence within the hinge region is replaced with an additional Glycine-Serine linker. The EPKSC sequence is not required for the VHH-CH3 fusions of the invention since this antibody format does not comprise a light chain. Again, each construct contained an additional VHH fused to the C-terminus of one CH3 domain, and KiH modifications in the CH3 domains to force heterodimerisation.

The present inventors then performed analysis of aggregation by Western Blot using non-reduced samples. This time, in contrast, the present inventors saw aggregation in the VHH-CH3 fusions (of the present invention), when utilizing either the truncated ΔCH2-hinge (SEQ ID NO: 8) or the truncated G1/G3:PAP-hinge (SEQ ID NO: 7). Moreover, the aggregation tendency was more pronounced in the VHH-CH3 fusions of the invention with the (truncated) G1/G3:PAP hinge (which is described in the literature to *increase* the stability of ΔCH2-IgGs) instead of the (truncated) ΔCH2-hinge, which contradicts the prior findings with ΔCH2 antibodies reported in the literature (Glaser et al., J. Biol. Chem, 2005, 280: 41494-503). The same experiment was performed using VHH with a different antigen specificity in the same formats, with no product being observed. It was further verified that these problems persisted with VHH-CH3 production regardless of the specificity of the VHHs, the presence of an additional C-terminal VHH, and the presence of the KiH mutations.

Hence, the hinge regions reported in literature for minibodies or ΔCH2-IgGs, respectively, surprisingly do not improve production of the VHH-CH3 fusions in the same manner, but rather increase the tendency of the VHH-CH3 constructs to aggregate.

Therefore, while the present inventors have now demonstrated that it is possible to produce the advantageous VHH-CH3 fusions of the present invention with typical linking sequences, e.g. using either the (truncated) ΔCH2-hinge or the (truncated) G1/G3:PAP hinge, the present inventors determined that production could be improved (e.g. for manufacture at the clinical scale or for the usage as RNA-encoded therapeutics).

### Example 2 - Improving the production of VHH-CH3 fusions

Without wishing to be bound by theory, the present inventors hypothesized that the additional flexibility of the hinge region in the VHH-CH3 fusions (due to the smaller size of the VHH compared to the Fab portion in ΔCH2-IgGs or the scFv moieties in minibodies) accounts for a larger molecular distance between the binding moiety and the CH3 domain as well as between the N-terminal VHHs themselves, such that the cysteines within the truncated G1/G3:PAP-hinge (SEQ ID NO: 7) or the truncated ΔCH2-hinge (SEQ ID NO: 8) do not form inter-chain, but inter-molecule disulphides, resulting in aggregation.

The initial design involved using the sequence as set forth in SEQ ID NO: 8 (i.e. GGGGSGGGGSDKTHTCPPCGGGSSGGGSG) as a linking sequence between the VHH and the KiH-CH3, where the lower hinge comprises two cysteines. The present inventors therefore replaced the cysteine residues in the hinge with serine residues in the "first generation" of improved constructs. The sequence of the linker in which cysteine residues in the hinge are replaced with serine residues is set forth in SEQ ID NO: 9 (i.e. GGGGSGGGGSDKTHTSPPSGGGSSGGGSG).

The present inventors then performed analysis of aggregation by Western Blot using non-reduced samples. The Western Blot analysis confirmed that, by removing the cysteines from the hinge region, unfavourable aggregation of the VHH-CH3 fusions was abolished completely in the first generation constructs and production was at a high level. It was further confirmed by biophysical analysis that the affinity of the VHH-CH3 fusions for their cognate targets are as expected (see Example 4, Table 1 below).

In addition, it was observed that by adapting the chain ratios (first polypeptide:second polypeptide) not only the amount of produced protein can be increased, but further benefits to production can be achieved with a most beneficial mass ratio of 1xKnob to 3xHole.

In minibodies and ΔCH2-IgGs (of the prior art), the hinge region contains the disulphide bridges in order to a) stabilize the overall molecule and b) try to create a covalent inter-chain connection resulting in an Isoform A molecule, instead of an Isoform B one (see e.g., Larson et al., J. Mol. Biol., 2005, 348: 1177-90). To avoid the formation of isoform B variants and simultaneously force the correct heterodimerisation by facilitating the covalent linkage of both CH3 domains, since it was desired not to form disulphide bonds between the hinge regions, the present inventors instead incorporated an asymmetrical disulphide bridge in the CH3 domain, along with the KiH mutations. These modifications meant that hinge cysteines were no longer needed at all within the VHH-CH3 fusion constructs of the invention. Hence, the issues a) and b) above seen with minibodies and ΔCH2 IgGs of the prior art were solved in a different way with the design of the present invention, by utilizing an inter-chain cysteine bridge in the CH3 domain instead of the hinge region.

### Example 3 - Further improvements to the VHH-CH3 fusion format

The present inventors also tested the VHH-CH3 fusions with a linking sequence containing significantly shorter GS-Linkers lacking any hinge region amino acids. The shorter GS-linkers have a sequence as set forth in SEQ ID NO: 10. These were termed "second generation" VHH-CH3 fusions. The present inventors determined that these more compact and more rigid second generation VHH-CH3 fusions would probably show significantly superior performance to the first generation VHH-CH3 fusions.

The second generation VHH-CH3 fusions did not show aggregates following analysis of aggregation by Western Blot using non-reduced samples. Further, the second generation VHH-CH3 fusions were produced similarly to the first generation variants exhibiting the cysteine to serine mutations. In particular, utilizing a shorter linker did not have a negative impact on the expression of the protein and the protein was fully functional and exhibited favourable biophysical properties (see Example 4 below). Of note, binding to the antigen of interest was not altered either (see Example 4, Table 1 below).

### Example 4 - Validation of VHH-CH3 fusions

To further validate the VHH-CH3 fusions, the present inventors performed affinity determination for each of the three generations of VHH-CH3 fusion described above (the initial constructs as well as the first and second generation constructs).

The tested VHH-CH3 fusions contained two N-terminal VHHs specific for a first antigen ("Antigen 1") and one C-terminal VHH specific for a second antigen ("Antigen 2)".

The functionality of each of the VHH-CH3 fusions were assessed by affinity determination to the Antigen 1 of each construct. All VHH-CH3 constructs were able to bind to the respective antigen, even in the presence of the aggregation seen during production of the initial constructs (see Example 1), underlining the functionality of the produced antibody constructs (Table 1).

**Table 1: Antigen affinity of the VHH-CH3 fusions with different generation of linkers/hinges**

| M: truncated ΔCH2-hinge (SEQ ID NO 8), G: truncated G1/G3:PAP hinge (SEQ ID NO 7), 1^{st} Gen: 1^{st} Generation of improved hinge (SEQ ID NO: 9), 2^{nd} Gen: 2^{nd} Generation of improved linker (SEQ ID NO: 10). VHH1-3: three different VHH-CH3 fusions with different target specificities, mediated by different VHHs. | | | | | | | | |
|---|---|---|---|---|---|---|---|---|
| **Affinity** | | **Antigen 1** | | | | **Antigen 2** | | |
| | | **Biolayer interferometry** | | | **On Cell EC50** | **Biolayer interferometry** | | |
| | | **KD [M]** | **ka [1/Ms]** | **koff [1/s]** | **[nM]** | **KD [M]** | **ka [1/Ms]** | **koff [1/s]** |
| **VHH1** | M | 1.084E-09 | 7.854E04 | 8.513E-05 | n.d. | n.d. | n.d. | n.d. |
| | G | 9.946E-10 | 6.975E04 | 6.937E-05 | n.d. | n.d. | n.d. | n.d. |
| | 1st Gen | 7.633E-10 | 5.207E05 | 3.975E-04 | 3.3 | 3.60E-08 | 4.851E05 | 1.748E-05 |
| | 2nd Gen | 1.549E-09 | 1.330E05 | 2.060E-04 | 1.3 | 5.786E-11 | 7.205E04 | 4.169E-06 |
| **VHH2** | M | 2.42E-10 | 1.06E+05 | 2.44E-05 | n.d. | n.d. | n.d. | n.d. |
| | G | 1.10E-09 | 8.13E+04 | 8.92E-05 | n.d. | n.d. | n.d. | n.d. |
| | 2nd Gen | 2.037E-11 | 1.128E05 | 2.298E-06 | 23.5 | 1.551E-10 | 2.250E05 | 3.488E-05 |
| **VHH3** | M | <1.0E-12 | 1.15E+05 | <1.0E-07 | n.d. | n.d. | n.d. | n.d. |
| | G | <1.0E-12 | 1.18E+05 | <1.0E-07 | n.d. | n.d. | n.d. | n.d. |
| | 2nd Gen | <1.0E-12 | 9.542E04 | <1.0E-07 | 1.9 | 3.593E-10 | 7.074E04 | 2.541E-05 |

To further validate the VHH-CH3 fusions, the present inventors performed biophysical characterisations of the first and second generation variants.

DLS (dynamic light scattering), SEC (size-exclusion chromatography) and MS (mass spectrometry) were all conducted, and all proved that the VHH-CH3 fusion as a whole behaves monomeric and monodisperse. Furthermore, NanoDSF (Nano differential scanning fluorimetry) underlined the favourable stability of these VHH-CH3 fusions (Table 2).

**Table 2: Biophysical characterization of VHH-CH3 fusions.**

| SEC purity, DLS results and thermal denaturation values determined by NanoDSF are depicted for first and second generation VHH-CH3 fusions. | | | | | |
|---|---|---|---|---|---|
| **Biophysical Properties** | | **SEC purity [%]** | **Cumulant Radius [nm]** | **Cumulant PDI** | **Tm value [°C]** |
| **VHH1** | 1st Gen | 97.8 | 5.59 | 0.02 | 53.47 |
| | 2nd Gen | 99.5 | 4.41 | 0.08 | 59.64 |
| **VHH2** | 2nd Gen | 96.8 | 4.09 | 0.03 | 56.85 |
| **VHH3** | 2nd Gen | 99.9 | 4.17 | 0.00 | 75.51 |

Further, the present inventors tested binding affinities of several VHH-CH3 fusions to their respective target antigens by FACS (fluorescence-activated cell sorting) and found that the binding behaviours were as expected (see Table 1). The present inventors tested three different VHHs (termed VHH1, VHH2 and VHH3) in the second generation VHH-CH3 fusions format as described above. The present inventors also tested one of these VHHs in the first generation VHH-CH3 fusion format (VHH1 hinge C -> S).

It was determined that all tested VHH-CH3 fusions were able to target both CHO and tumour cells expressing the target antigen of the VHH-CH3 fusions with high affinity and specificity (Figure 2). The use of the shortened linker in the second generation VHH-CH3 fusion format had no negative impact on cell binding as compared to the first generation VHH-CH3 fusion format containing the same VHH domain. Hence, these experiments confirmed that all tested constructs were fully functional.

Thus, FACS experiments showed that the VHH-CH3 fusions of the present invention were able to bind to target-positive cells, which underlines the full functionality of the VHH-CH3 fusions.

Moreover, the present inventors assessed binding affinity of the same 4 VHH-CH3 fusions, as well as a control antibody with a full-length Fc, to FcRn. As expected, the VHH-CH3 fusions did not interact with FcRn, while the antibody with a full-length Fc did.

### Discussion

The present inventors have generated an antibody fragment that combines the beneficial properties of minibodies or ΔCH2 IgGs with the small size and versatility of a VHH. This entirely novel antibody-related immunoglobulin domain-comprising class of constructs, "VHH-CH3 fusions", provides a functional antibody format.

It was further demonstrated that the VHH-CH3 fusions exhibiting cysteine residues in the hinge region show an elevated tendency to aggregate, which is unfavourable for large-scale production and would impede the use as an mRNA-based drug. To address this, the present inventors removed the capability of the linking sequences in the polypeptide chains of VHH-CH3 fusions to form disulphide bonds, as well as introducing a modification that enhances the dimerization of the polypeptide chains via forming a disulphide bond between the CH3 domains therein. The VHH-CH3 fusions with cysteine-free hinge regions showed production levels which were suitable for large-scale manufacture for clinical use and also exhibited biophysical properties needed to be alternatively used as an mRNA-encoded drug.

The present inventors also tested VHH-CH3 fusions with significantly shorter G-S linkers lacking any hinge region amino acids. These second generation VHH-CH3 fusions did not show aggregates and were produced as efficiently as the previous variants exhibiting the cysteine to serine mutations.

FACS experiments showed that all of the tested VHH-CH3 fusions were able to bind to target positive cells. This underlines the full functionality of the VHH-CH3 fusions.

Thus, this work provides new, small bispecific antibodies with deep tumour penetration and intermediate half-life.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

Various preferred features and embodiments of the present invention will now be described with reference to the following numbered paragraphs:
1. A construct comprising:
   a) a first polypeptide comprising one or more constant immunoglobulin domains and one or more variable immunoglobulin domains N-terminal of the constant immunoglobulin domains, wherein all of the constant immunoglobulin domains in the first polypeptide are constant heavy 3 (CH3) domains, and all of the variable immunoglobulin domains N-terminal of the CH3 domains are single domain variable heavy immunoglobulin (VHH) domains.
2. The construct of paragraph 1, further comprising:
   b) a second polypeptide comprising one or more constant immunoglobulin domains and one or more variable immunoglobulin domains N-terminal of the constant immunoglobulin domains, wherein all of the constant immunoglobulin domains in the second polypeptide are CH3 domains, and all of the variable immunoglobulin domains N-terminal of the CH3 domains are VHH domains.
3. The construct of paragraph 1 or 2, wherein the first polypeptide comprises a single CH3 domain and the second polypeptide, where present, comprises a single CH3 domain.
4. The construct of any one of paragraphs 1 to 3, wherein the first polypeptide comprises a single VHH domain N-terminal of the CH3 domains and the second polypeptide, where present, comprises a single VHH domain N-terminal of the CH3 domains.
5. The construct of any one of paragraphs 1 to 4, wherein, in the first polypeptide, the polypeptide sequence that is C-terminal of the CH3 domains comprises a binding moiety or the cognate of a binding moiety.
6. The construct of any one of paragraphs 2 to 5, wherein one or more of the CH3 domains of the first polypeptide and/or one or more of the CH3 domains of the second polypeptide comprise a modification that enhances the formation of the construct comprising the first and second polypeptide.
7. The construct of any one of paragraphs 2 to 6, wherein one or more of the CH3 domains of the first polypeptide and/or one or more of the CH3 domains of the second polypeptide comprise a modification that enhances the affinity between the first and second polypeptides.
8. The construct of any one of paragraphs 2 to 7, wherein one or more of the CH3 domains of the first polypeptide and/or one or more of the CH3 domains of the second polypeptide comprise a modification that reduces steric hindrance to the formation of the construct comprising the first and second polypeptide.
9. The construct of any one of paragraphs 2 to 8, wherein one or more of the CH3 domains of the first polypeptide and one or more of the CH3 domains of the second polypeptide are associated via a 'knobs-into-holes' modification.
10. The construct of any one of paragraphs 2 to 9, wherein the construct comprising the first polypeptide and the second polypeptide is a heterodimer.
11. The construct of any one of paragraphs 2 to 10, wherein a CH3 domain of the first polypeptide forms a disulphide bond with a CH3 domain of the second polypeptide.
12. The construct of any one of paragraphs 2 to 11, wherein:
   a) the sequence of the first polypeptide between the CH3 domains of the first polypeptide and the VHH domains located N-terminal of the CH3 domains in the first polypeptide consists of a first linking sequence; and
   b) the sequence of the second polypeptide between the CH3 domains of the second polypeptide and the VHH domains located N-terminal of the CH3 domains in the second polypeptide consists of a second linking sequence.
13. The construct of paragraph 12, wherein the first linking sequence does not form a disulphide bond with the second linking sequence.
14. The construct of paragraph 12 or 13, wherein each of the first and second linking sequences consists of a Glycine-Serine (GS) linker.
15. The construct of any one of paragraphs 12 to 14, wherein each of the first and second linking sequences comprises as few as 30, 29, 28, 27, 26, 25, 24, 23, 22, 21, 20, 19, 18, 17, 16, 15, 14, 13, 12, 11, 10, 9, 8, 7, 6, 5, 4, 3, 2, 1 or 0 amino acids.
16. The construct of any one of paragraphs 12 to 15, wherein each of the first and second linking sequences consists of 2 to 5 amino acids.
17. One or more nucleic acid sequences capable of expressing the construct according to any one of paragraphs 1 to 16, the first polypeptide as defined in any one of paragraphs 1 to 16, or the second polypeptide as defined in any one of paragraphs 2 to 16.
18. A nucleic acid particle comprising the construct or nucleic acid sequences according to any one of paragraphs 1 to 17.
19. A cell comprising the construct, nucleic acid sequences or nucleic acid particle according to any one of paragraphs 1 to 18.
20. A composition comprising the construct, nucleic acid sequences, nucleic acid particle or cell of any one of paragraphs 1 to 19.
21. An *in vitro* method comprising contacting a cell with the construct, nucleic acid sequences, nucleic acid particle, cell or composition of any one of paragraphs 1 to 20.
22. The construct, nucleic acid sequences, nucleic acid particle, cell or composition of any one of paragraphs 1 to 20 for use in a method of therapy or a diagnostic method.
23. A method for making a construct, wherein the method comprises:
   (i) providing a first polypeptide comprising one or more constant immunoglobulin domains and one or more variable immunoglobulin domains N-terminal of the constant immunoglobulin domains, wherein all of the constant immunoglobulin domains in the polypeptide are CH3 domains, wherein all of the variable immunoglobulin domains N-terminal of the CH3 domains are VHH domains, and wherein the sequence between the CH3 domains and the VHH domains located N-terminal of the CH3 domains consists of a first linking sequence;
   (ii) providing a second polypeptide comprising one or more constant immunoglobulin domains and one or more variable immunoglobulin domains N-terminal of the constant immunoglobulin domains, wherein all of the constant immunoglobulin domains in the polypeptide are CH3 domains, wherein all of the variable immunoglobulin domains N-terminal of the CH3 domains are VHH domains, and wherein the sequence between the CH3 domains and the VHH domains located N-terminal of the CH3 domains consists of a second linking sequence; and
   (iii) forming a construct comprising the first and second polypeptide by forming a disulphide bond between a CH3 domain of the first polypeptide and a CH3 domain of the second polypeptide, without forming a disulphide bond between the first linking sequence and the second linking sequence.

## Claims

1. A construct comprising:
a) a first polypeptide comprising one or more constant immunoglobulin domains and one or more variable immunoglobulin domains N-terminal of the constant immunoglobulin domains, wherein all of the constant immunoglobulin domains in the first polypeptide are constant heavy 3 (CH3) domains, and all of the variable immunoglobulin domains N-terminal of the CH3 domains are single domain variable heavy immunoglobulin (VHH) domains.

2. The construct of claim 1, further comprising:
b) a second polypeptide comprising one or more constant immunoglobulin domains and one or more variable immunoglobulin domains N-terminal of the constant immunoglobulin domains, wherein all of the constant immunoglobulin domains in the second polypeptide are CH3 domains, and all of the variable immunoglobulin domains N-terminal of the CH3 domains are VHH domains.

3. The construct of claim 1 or 2, wherein the first polypeptide comprises a single CH3 domain and the second polypeptide, where present, comprises a single CH3 domain.

4. The construct of any one of claims 1 to 3, wherein the first polypeptide comprises a single VHH domain N-terminal of the CH3 domains and the second polypeptide, where present, comprises a single VHH domain N-terminal of the CH3 domains.

5. The construct of any one of claims 1 to 4, wherein, in the first polypeptide, the polypeptide sequence that is C-terminal of the CH3 domains comprises a binding moiety or the cognate of a binding moiety.

6. The construct of any one of claims 2 to 5, wherein one or more of the CH3 domains of the first polypeptide and/or one or more of the CH3 domains of the second polypeptide comprise a modification that enhances the formation of the construct comprising the first and second polypeptide.

7. The construct of any one of claims 2 to 6, wherein a CH3 domain of the first polypeptide forms a disulphide bond with a CH3 domain of the second polypeptide.

8. The construct of any one of claims 2 to 7, wherein:
a) the sequence of the first polypeptide between the CH3 domains of the first polypeptide and the VHH domains located N-terminal of the CH3 domains in the first polypeptide consists of a first linking sequence; and
b) the sequence of the second polypeptide between the CH3 domains of the second polypeptide and the VHH domains located N-terminal of the CH3 domains in the second polypeptide consists of a second linking sequence.

9. One or more nucleic acid sequences capable of expressing the construct according to any one of claims 1 to 8, the first polypeptide as defined in any one of claims 1 to 8, or the second polypeptide as defined in any one of claims 2 to 8.

10. A nucleic acid particle comprising the construct or nucleic acid sequences according to any one of claims 1 to 9.

11. A cell comprising the construct, nucleic acid sequences or nucleic acid particle according to any one of claims 1 to 10.

12. A composition comprising the construct, nucleic acid sequences, nucleic acid particle or cell of any one of claims 1 to 11.

13. An *in vitro* method comprising contacting a cell with the construct, nucleic acid sequences, nucleic acid particle, cell or composition of any one of claims 1 to 12.

14. The construct, nucleic acid sequences, nucleic acid particle, cell or composition of any one of claims 1 to 12 for use in a method of therapy or a diagnostic method.

15. A method for making a construct, wherein the method comprises:
(i) providing a first polypeptide comprising one or more constant immunoglobulin domains and one or more variable immunoglobulin domains N-terminal of the constant immunoglobulin domains, wherein all of the constant immunoglobulin domains in the polypeptide are CH3 domains, wherein all of the variable immunoglobulin domains N-terminal of the CH3 domains are VHH domains, and wherein the sequence between the CH3 domains and the VHH domains located N-terminal of the CH3 domains consists of a first linking sequence;
(ii) providing a second polypeptide comprising one or more constant immunoglobulin domains and one or more variable immunoglobulin domains N-terminal of the constant immunoglobulin domains, wherein all of the constant immunoglobulin domains in the polypeptide are CH3 domains, wherein all of the variable immunoglobulin domains N-terminal of the CH3 domains are VHH domains, and wherein the sequence between the CH3 domains and the VHH domains located N-terminal of the CH3 domains consists of a second linking sequence; and
(iii) forming a construct comprising the first and second polypeptide by forming a disulphide bond between a CH3 domain of the first polypeptide and a CH3 domain of the second polypeptide, without forming a disulphide bond between the first linking sequence and the second linking sequence.
